# EUROPEAN PATENT SPECIFICATION

(11) **EP 4 665 305 B1**
(45) Date of publication and mention of the grant of the patent: **28.10.2026**
(21) Application number: 24706545.1
(22) Date of filing: 14.02.2024
(51) Int. Cl.: A61K 9/12, A61K 9/06, A61K 31/787, A61K 47/34, A61P 35/00, A61P 41/00, C08G 18/38, C08G 18/48, C08G 18/73, A61K 31/337, A61K 33/243

(54) **AEROSOL COMPOSITION**
AEROSOLZUSAMMENSETZUNG
COMPOSITION D'AÉROSOL

(30) Priority: 15.02.2023 NL 2034156
(43) Date of publication of application: 24.12.2025
(73) Proprietor: UPyTher B.V., 5612 AP Eindhoven (NL)
(72) Inventor: FRANSEN, Peter Paul Kathleen Hubert, 5631 JT Eindhoven (NL); DANKERS, Patricia Yvonne Wilhelmina, 5707 LH Helmond (NL); BRAET, Helena Anna, 8055 Zürich (CH); REMAUT, Katrien Free, 9000 Gent (BE); DE SMEDT, Stefaan Cornelis, 9030 Gent (BE)
(74) Representative: Kilburn & Strode LLP
(86) International application number: PCT/NL2024/050073
(87) International publication number: WO 2024/172649

(56) References cited:
- WO-A1-2014/185779
- WO-A1-2019/016208
- MAARTEN H. BAKKER ET AL: "Cholesterol Modification of an Anticancer Drug for Efficient Incorporation into a Supramolecular Hydrogel System", MACROMOLECULAR RAPID COMMUNICATIONS, vol. 39, no. 17, 28 May 2018 (2018-05-28), DE, pages 1800007, XP055506274, ISSN: 1022-1336, DOI: 10.1002/marc.201800007
- BAKKER MAARTEN H. ET AL: "Supporting Information - Cholesterol Modification of an Anticancer Drug for Efficient Incorporation into a Supramolecular Hydrogel System", MACROMOLECULAR RAPID COMMUNICATIONS, vol. 39, no. 17, 28 May 2018 (2018-05-28), DE, pages 1800007, XP093064910, ISSN: 1022-1336, Retrieved from the Internet <URL:https://onlinelibrary.wiley.com/action/downloadSupplement?doi=10.1002%2Fmarc.201800007&file=marc201800007-sup-0001-S1.pdf> [retrieved on 20230718], DOI: 10.1002/marc.201800007
- ROXANNE E. KIELTYKA ET AL: "Mesoscale Modulation of Supramolecular Ureidopyrimidinone-Based Poly(ethylene glycol) Transient Networks in Water", JOURNAL OF THE AMERICAN CHEMICAL SOCIETY, vol. 135, no. 30, 31 July 2013 (2013-07-31), pages 11159 - 11164, XP055161854, ISSN: 0002-7863, DOI: 10.1021/ja403745w

## Description

### Technical Field

The present invention relates to an aerosol composition comprising a UPy-PEG polymer and a carrier for use in treating diseases of the peritoneal cavity wherein the aerosol is administered by nebulization. The invention also relates to a drug nebulizing device for such use.

### Background

### Peritoneal cavity

The peritoneum lines the peritoneal cavity and is the largest and most complex serous membrane of the human body. It is a three dimensional organ covering the intra-abdominal (visceral peritoneum) organs and the abdominal wall (parietal peritoneum) including the interior surface of the diaphragm. It consists of a monolayer of mesothelial cells supported by a basement membrane and multiple layers of connective tissue with a total thickness of 90 µm.

The visceral peritoneum, also covering the mesentery, forms a continuous layer with the parietal peritoneum, which lines the abdominal wall and pelvic cavities. It can be seen as a large sac that tethers and covers the abdominal organs but still enables considerable mobility. The peritoneal membrane has a surface area of 1.5-2 m², similar to the skin, but is much larger if all microvilli are taken into account which is important for the role of the peritoneum as a transport barrier in intraperitoneal chemotherapy.

The peritoneal cavity is the largest serosal sac and fluid-filled cavity and contains 150-200 ml of fluid. Just like other serous membranes such as the pleura, the peritoneum secretes serous fluid (nearly 50 ml per day). The function of the peritoneum and the peritoneal fluid is to reduce friction between intraabdominal organs and the abdominal wall. It is important in the host's defense against intraabdominal infection as it is a barrier to infectious agents and is ripe with innate and adaptive immune cells.

The peritoneal cavity is an established route for the administration of drugs, such as peritoneal dialysis and painkillers after laparoscopy. Intraperitoneal instillation of anticancer drugs is used to treat peritoneal abdominal cancers. Several structures, including the peritoneal fluid, mesothelium, interstitium and blood vessel wall together compose a complex diffusion barrier: the peritoneal-plasma barrier. This means that after intraperitoneal administrations, drugs have to cross various different structures to get to the blood circulation. Vice versa, this barrier also inhibits obtaining effective intraperitoneal concentrations with systemic chemotherapy that is administered to the bloodstream.

The peritoneal cavity is a common injection site but its use as a common route of administration is hindered by several obstacles:
- The peritoneal cavity has a very irregular and complex topology with some areas that are difficult to access, especially after the formation of adhesions.
- The peristaltic motion of the gastrointestinal organs or mobility of the body cause internal movement.
- It is the largest fluid-filled cavity in the human body and requires large volumes for effective distribution which can be painful and harmful.

### Peritoneal adhesions

Peritoneal adhesions are pathological fibrous connections between surfaces in the abdominal cavity and are a significant cause of morbidity and mortality. They can develop after more than 50% of all abdominal operations, cause postoperative pain and intestinal obstructions and often require complicated repeat surgeries.

Adhesions formation starts with a local injury to the peritoneum, physically caused by surgery and injury or by irritation due to drug exposure, and is followed by an inflammatory response. The cell monolayer on tissues is damaged thereby leaving the tissues exposed to fibrin deposition. Normally, fibrinolytic processes remove the formed fibrin fibers within a few days, but if fibrin generation on the wound surface is faster than fibrinolysis, adhesions can form. Fibroblasts can then attach to the peritoneal surface to remodel the formed structure into a dense fibrotic structure. These processes occur within the first days to a week after injury.

After damaging the peritoneal tissue, fibrin deposition and matrix formation are seen within 12 hours, but it takes more than 7 days for new mesothelial tissue to form. Once fibrin deposits have been formed, the tissues are swollen and organs are closer to one another compared to normal. The fibrin deposits then act as bridges between the opposing tissues. In order to prevent the formation of adhesions, this post-inflammatory process must be halted.

In general, the prevention of adhesions relies on several different strategies: surgical techniques (laparoscopy vs open surgery), pharmacological agents, and physical barriers. The latter two inhibit the post-inflammatory process and are discussed below.

### Physical anti-adhesion barriers to prevent peritoneal adhesions

To prevent the formations of adhesions, anti-adhesions barriers are used which can come in various forms: solid membranes, gels and liquids. Solid membranes are placed directly on sites at risk of developing adhesions, but have to be fixated to the tissues by for example suturing. The fixation itself can in fact stimulate adhesion formation. Injectable hydrogels are promising devices because they can be administered as a liquid and adapt to the topography of the environment as the gel is formed. As a result, these types of materials are interesting for laparoscopic procedures or tissue areas that are difficult to access. Liquid solutions, mostly viscous polysaccharide polymers, can be poured or sprayed directly at the end of the operation. They can leak from the surgical site without adhering to the affected areas. Also, the peritoneum can become irritated by the large volumes of liquid.

Anti-adhesive barrier materials primarily do not actively interfere with the physiological processes that steer adhesion formation, such as coagulation, inflammation and wound healing. Instead they act as a physical barrier to separate the injured surfaces of the tissues, giving them time to heal without the formation of fibrinous structure and prevent the infiltration by fibroblasts. Ideally, these materials remain inert and are slowly resorbed and degraded by the human body without causing an extensive foreign body reaction. The device should be easy to use during laparoscopy or open surgery, uniformly cover the affected peritoneum, and remain *in situ* in the complex geometry of tissues during the healing process (days to weeks).

Limitations of existing devices are difficulty in handling and administration, and a brief residence time in the peritoneal cavity. A problem that often occurs is predicting the size and shape of the area with potential adhesions that needs to be covered with the anti-adhesive barrier. Surgeons either want a material that is easy to apply to cover discrete areas of injury or a material that can cover the entire peritoneum with a homogenous coating to protect unforeseen areas of injury.

Injectable hydrogels have the potential to collectively fulfill these requirements. As a result of physical cross-links, these materials form hydrogels inside the body without the need for organic solvents, chemical cross-linking agents or external devices (i.e. UV light). However, they require precise control over conflicting material properties such as viscosity, sol-gel transition, gelation time, and degradation rate. Highly viscous materials, with a high degree of cross-linking, resist fast degradation but are difficult to apply and unable to cover all surfaces. Very liquid materials can be injected in any geography, are minimally invasive to apply, and distribute freely but have no long term adhesion preventive effect. Furthermore, control over the sol-gel transition, and gelation time enables the surgeon to apply and fixate the material to a specific area of interest without the risk of rapid leakage.

### Pharmacological approach for adhesion prevention

In order to prevent adhesions, also pharmacological approaches can be used. A wide variety of drug compounds target different mechanisms that occur during the formation of adhesions.

| **Therapeutic targets** | **Drug category** | **Examples** |
|---|---|---|
| Reduce inflammation | Steroidal anti-inflammatory drugs | Progesterone, hydrocortisone, prednisone, estradiol, dexamethasone |
| | Non-steroidal anti-inflammatory drugs | Ibuprofen, flurbiprofen, tolmetin, nimesulide, indomethacin |
| | Proinflammatory cytokine inhibitors | Antibodies to TGF-β1 |
| | Antihistamine | Diphenhydramine, promethazine |
| | Free radical scavengers | Melatonin, vitamin E, superoxide dismutase |
| Prevent fibrin clot formation | Anticoagulants | Heparin, ancrod, warfarin, coumarin |
| Stimulate fibrinolysis | Proteolytic agents | Streptokinase, urokinase, pepsin, trypsin, tissue-type plasminogen activator, neurokinin 1 receptor antagonist |
| Inhibit fibroblast proliferation | Antiproliferative agents | Mitomycin C, 5-FU, doxorubicin, oxaliplatin, camptothecin |

Many of these anti-adhesive drugs are administered systemically and high amounts are needed. The absorption and diffusion properties of the mesothelium make it difficult to deliver these agents in a localized manner, especially in the peritoneum; Locally administered compounds suffer rapid clearance from the peritoneal cavity. To reduce possible adverse effects, pharmaceutical compounds can be added to material coatings or carriers to enable a local drug release, which is gradual and prolonged to prevent rapid wash-out from the affected area. And vice versa, whereas a pristine physical barrier only acts on a local site, a drug-loaded material is expected to have a more effective widespread anti-adhesion effect.

The ideal anti-adhesive device is able to combine two functions: control the drug release to provide sustained drug exposure and provide a physical barrier function. Additionally, the device material must be intrinsically inert, meaning that the material itself does not provoke adhesions that can negate the positive therapeutic effect of the pharmacological agents.

### Anti-adhesion polymer materials

Natural and synthetic polymers are used to design materials for the prevention of adhesions. Natural polymers closely resemble the biopolymers already present in the human body and in general are degraded quickly.

*Hyaluronic acid* - High molecular hyaluronic acid has a demonstrated anti-adhesive effect and can act as a physical barrier and promote the dissolution of fibrin. It also allows mesothelial cells to proliferate and promote the wound healing process. However, hyaluronic acid is rapidly degraded and absorbed *in vivo* and does not distribute uniformly after application.

*Cellulose* - Cellulose is the main component of two widely used devices for the prevention of adhesions: Interceed and Seprafilm. The potential of cellulose in adhesion prevention is demonstrated in multiple preclinical studies. However, its effectiveness is limited by the presence of peritoneal fluid and blood, which drastically limits the clinical use.

*Dextran -* In comparison to other natural polymer materials, dextran degrades more slowly. It is frequently employed as anti-adhesion material because it stimulates fibrinolysis and thrombolysis Dextran can enhance the expression of pro-inflammatory factors which may lead to worsened adhesion formation instead of suppressing it. Moreover, dextran can also provoke some side effects: anaphylactic reaction after intraperitoneal administration has been reported, also after removal of the material. Other adverse effects are liver dysfunction or labial oedema. Hyskon, by Medisan Pharmaceuticals, is a dextran-based anti-adhesive product that provokes local and systemic side effects and has shown insufficient evidence of clinical effectiveness.

*Chitosan* - Chitosan has a longer degradation time in the body than other biomaterials, and is therefore used to design long-acting devices. It has outstanding properties in blood clotting, antibacterial activity, and wound healing. It is more effective in hemostasis than in adhesion prevention, probably because pro-inflammatory factors such as TNF-a are upregulated. In addition, the solubility of chitosan is significantly decreased when the pH is higher than 6.5, which also limits its anti-adhesion ability. In the peritoneal cavity the pH ranges between 6.5-8.5 and therefore hinder the effectiveness of chitosan.

*Icodextrin -* A 4% icodextrin solution is marketed as Adept. It can flow freely as a liquid and can therefore be used for intraoperative or postoperative instillation to prevent adhesions. Side-effects are pain, abdominal discomfort and abdominal distention.

Synthetic polymers on the other hand are more resistant to degradation and their properties can be tuned easily. Most synthetic polymers have a lower cost compared to natural polysaccharides that undergo complicated extraction and purification processes. However, when using synthetic materials it is important to avoid a severe foreign body reaction.

*Polyethylene glycol (PEG)* - PEG has excellent biocompatibility and is often used in adhesion prevention devices and pharmaceutical products. As an anti-adhesive material, it can cover the injured tissues and prevent contact with other organs, prevent the adsorption of proteins because of its non-fouling properties. Also, because of the osmotic pressure it stimulates the production of peritoneal fluid that lubricates the tissues and inhibits inflammation.

Spraygel is a product that consists of two liquids that are mixed and sprayed through a nozzle and gelate on the surface of the organs. However, this system is not compatible with conventional laparoscopy because of the CO₂ that is used which hampers gel formation. Other systems such as FocalGel require a light source to induce *in situ* cross-linking. In general, it is apparent that the lack of an effective administration technique and cross-linking approach hampers the use of PEG, a material with promising intrinsic anti-fouling properties, in anti-adhesive devices. The invention of this patent application teaches the use of UPy-functionalized PEG-polymers that can be administered easily and cross-link *in situ* to form a semi-solid barrier material. The applicants propose that the invention overcomes the current limitations of the state of the art.

*PLA* (polylactic acid) - PLA-based barriers can not only act as physically separate injured areas and nearby normal sites, but are also good drug carriers to perform the constant drug release behavior to inhibit adhesion formation. PLA-materials have a high melting point and excellent strength, but also have poor processability, flexibility and a high cost.

Many of the described products are withdrawn from the market due to concerns about their safety and effectiveness related to material-related problems described above.

### Pressurized intraperitoneal aerosol chemotherapy (PIPAC)

PIPAC is a recently developed intraperitoneal therapy. It is used to manage disease in patients with extensive, inoperable peritoneal carcinomatosis. With this technique, a low dose of chemotherapeutic agent is administered in the form of an aerosol to the peritoneal cavity by a laparoscopic approach. In theory PIPAC should result in: (1) high intraperitoneal concentrations, but low systemic concentrations and low systemic toxicity, (2) a homogeneous distribution, and (3) deep local tissue penetration. The latter two advantages are achieved by using the advantages of a fine floating aerosol and increased intraabdominal pressures of 12 mmHg. The addition of electrostatic precipitation to the aerosol could result in a greater tissue penetration of the chemotherapeutic agent (ePIPAC), thereby increasing the effectiveness of intraperitoneal chemotherapy.

Lurvink & Deenen et al. showed that the systemic exposure of oxaliplatin administered by ePIPAC was similar to the exposure after systemic chemotherapy (C_{max, systemic}: 1.61-1.92 µg/mL vs C_{max, ePIPAC}: 2.67-3.28 µg/mL; AUC_{max, systemic} 51.4-118.0 µg/mL·h vs. AUC_{max, ePIPAC} 49.0-59.5 µg/mL·h) This finding negates one of the proclaimed benefits of ePIPAC, namely a lower systemic drug exposure and consequently fewer toxic side effects. During PIPAC, the total drug amount is administered in 5 minutes. Therefore , in order to improve the effectiveness and safety of PIPAC, the drug absorption by the body has to be slowed down to prolong local exposure and decrease systemic drug exposure. Therefore there is an important technical need for a controlled release formulation that is compatible with aerosol administration.

### Hydrogels for aerosol administration

Braet, H. et al. teaches hydrogel formulations based on thermoreversible Pluronic polymers which can be administered by aerosol. An irreconcilable tradeoff is observed between controlled release properties and suitability for aerosolization: denser hydrogels (20% and 25% w/v Pluronic F127) are able to sustain nanoparticle release up to 30 hours, but cannot effectively be nebulized and vice versa. In order to improve these properties, alternative controlled release formulations are required.

Bakker et al. show sustained release of a cholesterol-modified mitomycin C from a pH-responsive supramolecular UPy-PEG hydrogel.

The present invention now provides an aerosol composition that fulfills the above requirements. Additionally, the invention overcomes current technical challenges regarding the cross-linking of PEG-based polymers, a material with excellent intrinsic anti-adhesive properties.

### Summary

According to a first aspect, the present invention relates to an aerosol composition comprising:
a) a UPy-PEG polymer having hydrophobic hard blocks covalently bonded with hydrophilic soft blocks wherein the hydrophobic hard blocks comprise 2-ureido-4[1H]-pyrimidinone (UPy) moieties and aliphatic spacers and the hydrophilic soft blocks comprise polyethylene glycol moieties; and
b) a pharmaceutically acceptable carrier

wherein the aerosol composition is characterized by
   - a viscosity of 1 to 500 mPa.s, preferably 1 to 100 mPa.s over a temperature range of 15 to 25 °C and a pH range of 8.5 to 14;
   - a viscosity of 1 to 20 mPa.s, preferably 1 to 5 mPa.s over a temperature range of 35 to 50 °C and a pH range of 8.5 to 14;
   - a viscosity of at least 100 mPa.s, preferably at least 1000 mPa.s over a temperature range of 35 to 40 °C and a pH range of 6 to 8.
wherein the viscosity is measured at shear rate 1 s⁻¹,
for use in a method of treatment or prevention of a disease of the peritoneal cavity, wherein the treatment comprises administration of the aerosol composition to the peritoneal cavity by nebulization.

According to a second aspect, the invention relates to a drug nebulizing device for use in such a method of treatment or prevention for delivery of aerosolized pharmaceutical compositions into the peritoneal cavity.

The aerosol composition of the current invention overcomes the technical challenge described in the prior art as the material composition is suitable for aerosolization, but can remain *in situ* in the peritoneal cavity to prolong the drug release for a longer time, e.g. 7 days. Additionally, the aerosol composition enables precise control over conflicting material properties such as viscosity, sol-gel transition, gelation time, and degradation. These material properties strongly influence the ease of administration, handling and peritoneal distribution, but also the residence time in the peritoneal cavity.

### Definitions

With aerosol is meant a suspension of fine liquid droplets in a gas. Nebulization or aerosolization is the process of creating an aerosol from an aerosol composition.

With 2-ureido-4[1H]-pyrimidinone (UPy) moieties are meant moieties that can forms dimers through quadruple hydrogen bonding and are derived from:

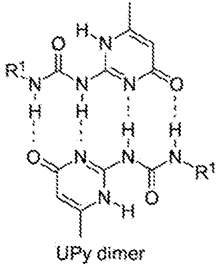

With aliphatic spacers are meant aliphatic hydrocarbon moieties, in particular alkyl moieties.

### Detailed Description

### Aerosol composition

As described above, the present invention provides an aerosol composition comprising a specific UPy-PEG polymer in a carrier which is characterized by a viscosity profile that makes it possible to administer the aerosol composition to the peritoneal cavity of a subject with conventional aerosol techniques. Once present in the peritoneal cavity, the viscosity of the hydrogel increases under the influence of the physiological parameters such as body temperature and pH.

### Viscosity

The requirement for developing an aerosol composition that can be administered to the peritoneal cavity is that the composition behaves as a liquid at non-physiological conditions but responds to an external trigger (change in pH or temperature) to switch to a solid material. In particular, the formulation is liquid at room temperature (15 to 25 °C) outside the body and solid-like or gel-like inside the peritoneal cavity at pH 6 to 8 and temperature of 35 to 40 °C.

Thus, the aerosol composition has:
a viscosity of 1 to 500 mPa.s, preferably 1 to 100 mPa.s, more preferably 1 to 50 mPa.s or even 1 to 25 mPa.s over a temperature range of 15 to 25 °C and a pH range of 8.5 to 14;
a viscosity of 1 to 20 mPa.s, preferably 1 to 5 mPa.s over a temperature range of 35 to 50 °C and a pH range of 8.5 to 14; and
a viscosity of at least 100 mPa.s, preferably at least 1000 mPa.s over a temperature range of 35 to 40 °C and a pH range of 6 to 8.

Viscosity is determined at 20°C by rotational rheology measurements using a conical plate geometry, with a fixed distance of 0.101 mm wherein shear viscosity is recorded as function of shear rate with 600 µL of liquid hydrogel solution applied onto the plate of the rheometer. The viscosity is measured at shear rate 1 s⁻¹.

At an elevated pH of at least 9, all aerosol compositions display Newtonian behavior with a constant viscosity for all shear rates. At physiological pH, the aerosol composition of the invention displays non-Newtonian behavior with an increase in viscosity at lower shear rates. The increase in viscosity, and resistance to flow, is the result of hydrogel network formation.

Preferably, the aerosol composition is further characterized by a storage modulus of 10 to 2000 Pa at a temperature range of 35 to 40 °C. The storage modulus is determined at 37°C by oscillatory rheology measurements using a conical plate geometry, with a fixed distance of 0.101 mm, wherein storage and loss moduli are recorded as function of strain (0.1-100 %, 10 points per decade, frequency = 1 Hz) and for frequency sweep measurements, storage and loss moduli are recorded as function of frequency (0.1-100 rad/s, 10 points per decade, strain = 1%) with 600 µL of hydrogel applied onto the plate of the rheometer.

### Carrier/concentration

The pharmaceutically active carrier is preferably an aqueous solution that may optionally contain a buffer. Preferably the pharmaceutically active carrier is phosphate buffered saline (PBS) buffer solution.

The carrier can also be used to adjust the pH of the hydrogel composition to obtain the viscosity profile as outlined above. Preferably the aerosol formulation has a pH of 8.5 to 12, more preferably at least 9, most preferably of 10 to 12.

The concentration of the UPy-PEG polymer in the aerosol composition is 0.5 to 20 wt.%, preferably 1 to 10 wt.%, more preferably 4 to 8 wt.%. The concentration of the UPy-PEG polymer also influences the viscosity.

The aerosol composition is prepared by dissolving the UPy-PEG polymer in the carrier. The carrier may be heated to a temperature of 30-80 °C to accelerate the dissolution. The pH of the composition can be adjusted by use of an appropriate buffer solution as the carrier. Alternatively, the pH can be adjusted to a value of at least 9, more preferably at least 10, after dissolving the UPy-PEG polymer, for instance by adding a sodium hydroxide solution.

### UPy-PEG

The composition comprises a supramolecular UPy-functionalized polyethylene glycol, a synthetic polymer that is generally regarded as biocompatible, and also effective in treating adhesions. However, its application in the peritoneal cavity is limited by difficulties in effective cross-linking to form a hydrogel material. The described invention comprises a supramolecular UPy-PEG-based material that can form physical cross-links after administration via aerosol. After administration, by for example a laparoscopic procedure, a continuous hydrogel layer coats the peritoneal surfaces.

Due to the properties of the material combined with an aerosol-administration technique, such as PIPAC, the material can spread to all tissues sites at risk of developing adhesions or tumors. It not only acts as an effective physical barrier to prevent the formation of adhesions, but can also serve as a drug depot to prolong drug exposure in the treatment of peritoneal adhesions or malignancies. The latter functionality is a result of the UPy-functionalization, which creates hydrophobic compartments for the retention of therapeutic agents.

According to an embodiment the UPy-PEG polymer is obtainable by the reaction of:
a compound having formula (I) 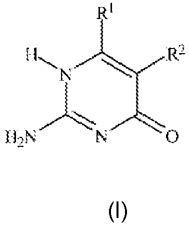 wherein
   R¹ is independently selected from the group consisting of hydrogen and C₁-C₂₀ alkyl,
   R² is independently selected from the group consisting of hydrogen and C₁-C₂₀ alkyl,
   wherein C₁-C₂₀ alkyl is optionally substituted by -OH,
a diisocyanate compound with formula OCN-R³-NCO
   wherein R³ is C₂-C₁₆ alkyl or C₄-C₁₆ alkenyl
optionally, an aliphatic spacer which is C₂-C₂₄ alkyl
and a polyethylene glycol.

According to a first preferred embodiment, in Formula (I), R¹ is preferably C₁-C₅ alkyl, more preferably methyl. R² is preferably H. Alkyl includes linear and branched alkyl, but is preferably linear alkyl.

In the diisocyanate compound, R³ is preferably C₄-C₁₀ alkyl. The diisocyanate compound is more preferably hexane diisocyanate (HDI).

The UPy-PEG polymer preferably has a ratio of hard blocks to soft blocks of 1:5 to 1:25 based on molecular weight. As described above, the UPy-PEG polymer comprises hydrophilic polyethylene glycol soft blocks, preferably having a molecular weight Mₙ of 5,000 to 30,000 Da, more preferably 10,000 to 20,000 Da. According to an embodiment, the PEG is a telechelic polymer which is hydroxy functional on both ends.

The UPy-PEG polymer in particular has a molecular weight Mₙ of 5,000 to 1,000,000 Da, preferably 10,000 to 100,000 Da, more preferably 10,000 to 50,000 Da. Molecular weights can be determined by end group titration or size exclusion chromatography.

A preferred group of UPy-PEG polymers is described in US8,628,789.

According to a second preferred embodiment, the UPy-PEG polymer is a chain-extended UPy-PEG polymer which is obtainable by a process wherein
a compound A with formula (I) 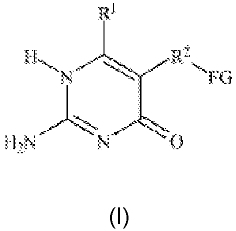 wherein
   R¹ is independently selected from the group consisting of hydrogen and C₁-C₂₀ alkyl,
   R² is C₁-C₂₀ alkyl, and
   FG is a functional group independently selected from OH and N(R¹)H
is reacted with
a diisocyanate compound B with formula OCN-R³-NCO
   wherein
   R³ is C₂-C₁₆ alkyl or C₄-C₁₆ alkenyl
   and
a polymer C with formula HO-P-OH
   wherein
   P is a polymeric group having a Mₙ of 250 to 50,000.

In Formula (I) of compound A, R¹ is preferably C₁-C₅ alkyl, more preferably methyl. R² is preferably C₁-C₅ alkyl, more preferably ethyl. FG in compound A is preferably OH.

In diisocyanate compound B R³ is preferably C₄-C₁₀ alkyl. The diisocyanate compound B is more preferably hexane diisocyanate (HDI).

HO-P-OH preferably represents a polyethylene glycol (PEG). The molecular weight Mₙ of the PEG is preferably 250 to 50,000 Da. The Mₙ can be determined by end group titration. Preferably the Mₙ is 5,000 to 30,000 Da, more preferably 15,000 to 25,000 Da. The polyethylene glycol is preferably telechelic with the reactive functional groups at chain ends.

The hydrophilic/hydrophobic behavior of the UPy-PEG polymer can be adjusted by adjusting the molar ratio's wherein compound A, diisocyanate compound B and polymer C are reacted. Preferably the molar ratio C:(A+B) is 1:1 to 1:15, more preferably 1:2-1:11.

The UPy-PEG polymer used according to the invention can also be represented as

[PEG-I-U-I-U-I]ₙ

wherein:
PEG is polyethylene glycol
I is a aliphatic diisocyanate
U is a hydrogen bonding UPy-moiety (formula I)
I and U indicate the building blocks of the UPy-PEG polymer. During the reaction where the UPy-PEG polymer is formed the isocyanate is converted to a urea or urethane bond.

A class of such UPy-PEG polymers is known from WO2014/185779 which is incorporated herein by reference.

The UPy-PEG polymer of the second preferred embodiment can be prepared by reacting compounds A, B and C in an appropriate solvent in the presence of an appropriate catalyst by methods known in the art, for example in solution or in the bulk using reactive extrusion. The process is preferably performed at a temperature between about 10 °C and about 140 °C, more preferably between about 20 °C and about 120 °C, and most preferably between about 40 °C and about 90 °C.

The process for the preparation of the polymer may be performed in the presence of a catalyst. Examples of suitable catalysts are known in the art and they promote the reaction between isocyanates and hydroxyl groups. Preferred catalysts include tertiary amines and catalysts comprising a metal. Preferred tertiary amines are 1,4-diazabicyclo[2.2.2]octane (DABCO) and 1,8-diazabicyclo[5.4.0]undec-7-ene (DBU). Preferred catalysts comprising a metal are tin(IV) compounds and zirconium(IV) compounds, preferably selected from the group consisting of tin(ll)octanoate, dibutyltin(IV)laurate and zirconium(IV)acetoacetate. Most preferably, the catalyst is dibutyltin(IV)laurate. The amount of catalyst is generally below about 1 wt.%, preferably below about 0.2 wt.% based on the total amount of reactants.

The process may be performed in the presence of a non-reactive organic solvent, wherein it is preferred that the amount of the non-reactive organic solvent is at least about 20 wt.%, more preferably at least about 40 wt.%, based on the total weight of the reaction mixture. It is also preferred that the reaction mixture does not comprise any inorganic solvents such as water.

Non-reactive solvents may be selected from non-protic polar organic solvents, preferably tetrahydrofuran, dioxane, N-methylpyrollidone, dimethylformamide, dimethylacetamide, dimethyl sulfoxide, propylene carbonate, ethylene carbonate and 2-methoxy-ethyl-acetate.

### Aerosol administration

According to the invention, the aerosol composition is used in the treatment of a disease of the peritoneal cavity, wherein the treatment comprises administration of the aerosol composition to the peritoneal cavity by nebulization. With nebulization or aerosolization is meant that the aerosol composition is administered in the form of fine droplets, as an aerosol. Once present in the peritoneal cavity the aerosol composition will form a gel.

Preferably the droplet size is less than 200 µm, preferably less than 150 µm, more preferably less than 100 µm. Droplet size can be determined by laser diffraction (see Braet et al.).

Different nebulization techniques are known to the skilled person. According to a preferred embodiment the nebulization is carried out under elevated pressure, in particular using a pressurized intraperitoneal chemotherapy (PIPAC) spray nozzle and a high pressure injector. More preferably, nebulization is carried out using electrostatic precipitation pressurized intraperitoneal chemotherapy (ePIPAC) treatment. In an embodiment, the treatment further comprises inducing an electrostatic field in the peritoneal cavity. Further, the treatment in particular comprises inducing a capnoperitoneum with carbon dioxide gas.

According to an aspect of the invention, the invention provides a method of forming an aerosol comprising nebulizing an aerosol composition of the invention, creating droplets of the aerosol composition having a droplet size of less than 200 µm, preferably less than 150 µm, more preferably less than 100 µm..

### Device

According to a further aspect of the invention, it comprises a drug nebulizing device for use in a method of treatment or prevention of a disease of the peritoneal cavity, wherein the device is adapted for delivery of aerosolized pharmaceutical compositions into the peritoneal cavity, wherein the device comprises:
a reservoir containing the aerosol composition according to the invention; optionally provided with means to control temperature; and
a spray nozzle; and optionally
means to create high pressurized aerosol; and optionally
means to create electrostatic field.

The invention thus also relates to a reservoir containing the aerosol composition according to the invention for use in a drug nebulizing device comprising the aerosol composition of the invention

### Disease

The aerosol composition of the invention is used for the prevention or treatment of a disease of the peritoneal cavity. With prevention is meant that the aerosol composition can be administered before the disease occurs, for instance in the prevention of metastasis. With treatment is meant that the aerosol composition decreases the disease or removes the disease all together.

According to a preferred embodiment, the disease comprises adhesions in the peritoneal cavity, in particular after surgical intervention, other treatments of the peritoneal cavity or peritoneal carcinomatosis.

According to a further preferred embodiment, the disorder comprises a peritoneal disease. With peritoneal diseases is meant diseases that occur in the peritoneal cavity. According to a preferred embodiment, the peritoneal diseases comprise peritoneal carcinomatosis, including primary tumors of the peritoneum such as peritoneal mesothelioma and primary peritoneal cancer, and peritoneal metastasis from tumors of other sites, including intraperitoneal origins, such as gastrointestinal and gyneacological cancer, including ovarian, and sarcoma, and extraperitoneal origin sites such as lung, skin and kidney cancers. The present invention encompasses treatment of cancers, but also prevention of recurrence of cancers, i.e. metastasis.

### Pharmaceutically active agent

The hydrogel composition of the invention allows for the incorporation of different pharmaceutically active agents that are known for the treatment or prevention of peritoneal diseases. The pharmaceutically active agent may be selected from antineoplastic drugs, chemotherapeutic agents, monoclonal antibodies, immunomodulating compounds, targeted therapies and combinations thereof .

According to an embodiment of the invention, the pharmaceutically active agent is selected from antineoplastic drugs and chemotherapeutic agents, in particular from mitomycin C, oxaliplatin, carboplatin, cisplatin, gemcitabine, 5-fluorouracil (5-FU), paclitaxel, docetaxel, irinotecan, doxorubicin and combinations thereof. According to another embodiment of the invention, the pharmaceutically active agent is selected from immunomodulating compounds such as TLR-agonists (imidazoquinolines), STING-agonists (cyclic dinucleotides) and combinations thereof. According to another embodiment of the invention, the pharmaceutically active agent is selected from targeted therapies such as ATR-inhibitors, PARP-inhibitors and combinations thereof. The hydrogel composition is also suitable for the combination of antineoplastic drugs and/or chemotherapeutic agents with targeted therapies.

One of the advantages of the invention is that the hydrogel composition may comprise both water soluble as well as less water soluble or water insoluble pharmaceutically active agents. The water insoluble active agents can be included in the hydrogel composition without the need of additives such as surfactants, DMSO or PEG400.

More hydrophobic active agents will have increased affinity with the hydrophobic compartments of the hydrogel and will be released at a slower rate than more hydrophilic active agent. The hydrogel composition thus enables a synergetic sequential combination therapy.

### Sustained release

As described above, one of the advantages of the invention is that the pharmaceutically active agent can be released in a controlled way. Thus the treatment of the invention comprises sustained release of the active agent. A preferred release rate is a rate of more than 80% in a time range of 2 to 30 hours for a hydrophilic active agent and of 2 to 30 days for a hydrophobic active agent.

This release rate can be determined by loading a model drug compound from a stock solution (30 mM in DMSO) to 0.3 mM in the hydrogel composition (precursor solution, transferring the drug loaded solution (100 µL) to a Millicell insert, placed in a 24-wells plate filled with PBS pH 7.4, 7.8 or 8.2 (600 µL), refreshing the PBS at set times and analyzing the removed PBS for drug content by UV absorbance wherein the release experiments are performed with n = 3. Other methods are LC-MS or ICP-MS. Such methods are known to a skilled person.

### Brief Description of the Drawings

Figures 1A-1C show viscosity as a function of shear rate for different formulations at different pH and temperature values.
Figure 2 shows a frequency and strain sweep of a UPy-PEG hydrogel (pH 7.4) at 37 °C before (pristine) and after (aerosol) aerosolization.
Figures 3 shows swelling and degradation of UPy-PEG hydrogels in PBS buffer (4A) and ascites (4B).
Figure 4 shows the viability of SKOV3-IP2 cells after 1, 2 and 4 days of incubation with different concentrations of 3 and 4 wt% UPy-PEG hydrogels determined by the MTT assay. A cell viability threshold of 90% is shown as reference (dashed line). Data are expressed as the average (n=3) and error bars represent the standard deviation.
Figure 5 shows thickness of gel layer and mass change of tissue samples after nebulization of UPy-PEG solutions in an *ex vivo* model of the peritoneal cavity. Data are expressed as the average (n=3) and error bars represent the standard deviation.
Figure 6 shows the cumulative release of paclitaxel from the paclitaxel nanocrystal (PNC) loaded UPy-PEG hydrogel in PBS + 0.2% Tween 80 (PBS-T) at pH 7.4 and cumulative release of cisplatin from the cisplatin nanoparticle (cisPt-NP) loaded UPy-PEG hydrogel in PBS at pH 7.4.
Figure 7 shows the viability of SKOV-3 IP2 cells following incubation with the PNC loaded gel or PNC suspension and following incubation with the cisPt-NP loaded gel or cisPt-NP suspension. At each time point, inserts containing the formulations were transferred to freshly seeded wells. Data are expressed as the average (n=3) and error bars represent the standard deviation and the viability of SKOV-3 IP2 cells.
Figures 8A and 8B show the cumulative release of TLR-agonist R848 (Fig 8A) and TLR-agonist MEDI9197 (Fig 8B) from 6 and 10 wt.% hydrogel formulations.
Figure 9 shows the cumulative release of monoclonal antibody ipilimumab from a 10 wt.% hydrogel formulation.

### References

Bakker et al.: Bakker, M. H. et al. Cholesterol Modification of an Anticancer Drug for Efficient Incorporation into a Supramolecular Hydrogel System. Macromol. Rapid Commun. 39, 1800007 (2018) Bastings et al.: Bastings, M. M. C. et al. A Fast pH-Switchable and Self-Healing Supramolecular Hydrogel Carrier for Guided, Local Catheter Injection in the Infarcted Myocardium. Advanced Healthcare Materials 3, 70-78 (2014)
Binda, M. M., Molinas, C. R., Bastidas, A. & Koninckx, P. R. Effect of reactive oxygen species scavengers, antiinflammatory drugs, and calcium-channel blockers on carbon dioxide pneumoperitoneum-enhanced adhesions in a laparoscopic mouse model. Surg. Endosc. Other Interv. Tech. 21, 1826-1834 (2007)
Braet, H. et al. : Braet, H. et al. Exploring high pressure nebulization of Pluronic F127 hydrogels for intraperitoneal drug delivery. European Journal of Pharmaceutics and Biopharmaceutics 169, 134-143 (2021))
De Clercq, K. et al. Genipin-crosslinked gelatin microspheres as a strategy to prevent postsurgical peritoneal adhesions: In vitro and in vivo characterization. Biomaterials 96, 33-46 (2016)
Egea, A. M., Aguayo Albasini, J. L., Carmona, G. Z. & Parido, P. P. Adhesion Response to Different Forms of Treating a Peritoneal Lesion: An Experimental Study in Rats. Dig. Surg. 12, 334-337 (1995)
Ersoy, E., Ozturk, V., Yazgan, A., Ozdogan, M. & Gundogdu, H. Comparison of the two types of bioresorbable barriers to prevent intra-abdominal adhesions in rats. J. Gastrointest. Surg. 13, 282-286 (2009)
Kieltyka et al.: J. Am. Chem. Soc. 2013, 135, 30, 11159-11164
Lurvink et al. : Lurvink, R. J. et al. Systemic Pharmacokinetics of Oxaliplatin After Intraperitoneal
Administration by Electrostatic Pressurized Intraperitoneal Aerosol Chemotherapy (ePIPAC) in Patients with Unresectable Colorectal Peritoneal Metastases in the CRC-PIPAC Trial. Ann Surg Oncol 28, 265-272 (2021)
Shariati, M. *et al.* Synergy between Intraperitoneal Aerosolization (PIPAC) and Cancer
Nanomedicine: Cisplatin-Loaded Polyarginine-Hyaluronic Acid Nanocarriers Efficiently Eradicate Peritoneal Metastasis of Advanced Human Ovarian Cancer. Cite This ACS Appl. Mater. Interfaces 12, (2020)
Van de Sande, L. et al.(1): Establishment of a rat ovarian peritoneal metastasis model to study pressurized intraperitoneal aerosol chemotherapy (PIPAC). BMC Cancer 19, 424 (2019)
Van de Sande, L. et al.(2): Electrostatic Intraperitoneal Aerosol Delivery of Nanoparticles: Proof of Concept and Preclinical Validation. Adv. Healthc. Mater. 2000655 (2020). doi: 1 0.1 002/adhm.202000655
Zhang, Z. et al. Biodegradable and thermoreversible PCLA-PEG-PCLA hydrogel as a barrier for prevention of post-operative adhesion. Biomaterials 32, 4725-4736 (2011).

### Example 1 Aerosol formulations of bifunctional UPy-PEG polymers

### 1.1 Materials and methods

### Polymer design & synthesis

The tested UPy-PEG polymers were the bifunctional telechelic UPy-PEG₁₀ₖ and UPy-PEG₂₀ₖ polymers as described in Kieltyka et al. They were synthesized as follows:
CDI-activation of PEG prepolymers: - Solid poly(ethylene glycol) with molecular weight 10 or 20 kDa (PEG; 1 eq, usually 2-5 grams) was added to a solution of 1,1-carbonyldiimidazole (CDI; 8 eq) in dichloromethane (10-15 mL/g PEG, depending on solution viscosity) and allowed to stir at room temperature for 8 hours. Excess CDI and imidazole were removed by precipitation by slowly diluting the dichloromethane solution with diethyl ether while stirring vigorously. The precipitating material was stirred for 10-20 minutes, then allowed to settle down for 10 minutes. Solid product was filtered off using vacuum filtration on a glass fritted filter followed by brief drying with nitrogen flow. Upon initial precipitation, the polymer was reprecipitated and lightly dried with nitrogen. The CDI-activated poly(ethylene glycol) was used immediately upon isolation of the solid polymer.

Diamine-termination of PEG prepolymers: - CDI-activated PEG polymer (1 eq; 1-4 g) was dissolved under a N₂ atmosphere in dichloromethane (15 mL/g PEG) and was added dropwise to a solution of 1,12-diaminododecane (6-8 eq) in chloroform over 30 minutes and was allowed to stir at room temperature for 8 hours. A small amount of methanol (~25 vol. %) was added to the reaction mixture to aid in the diamine solubility during precipitation and to facilitate removal. Excess 1,12-diaminododecane was then removed via precipitation from dichloromethane chloroform solutions into diethyl ether as performed in the previous step. This procedure was also performed twice. The isolated polymer was vacuum dried overnight. 1H NMR (400 MHz, CDCl₃): δ = 4.9 (2H, urethane), 4.2 (4H, next to urethane), 3.9-3.2 (4nH, PEG), 3.1 (4H, next to urethane), 2.7 (4H, next to amine), 2.0 (4H, broad, next to urethane), 1.5-1.1 (16H, hexyl spacer or 48H, dodecyl spacer) ppm.

UPy-functionalization with UPy-isocyanate: - Solid 2(6-isocyanatohexylaminocarbonylamino)-6-methyl-4[1H]pyrimidinone (2.5 eq) was added to a solution of solid diamine terminated-PEG (1 eq; 1-2 g) in a 1:1 mixture of dichloromethane and chloroform, and was stirred at room temperature for 16 hours. Excess UPy-isocyanate was trapped via addition of silica gel (3-5 g) and dibutyltindilaurate catalyst (1 drop) to the reaction mixture and heating for 2-3 hours at reflux temperature. Upon concentration of the filtered solutions to a noticeable viscosity increase, reprecipitation was performed by dilution with diethyl ether. Polymers were vacuum dried at 40 °C for 8 hours prior to structural characterization. 1H NMR (400 MHz, CDCl3): δ = 13.1 (2H, UPy), 11.8 (2H, UPy), 10.1 (2H, UPy), 5.8 (2H, UPy alkylidene), 4.9 (2H, urethane), 4.7 (2H, urea), 4.5 (2H, urea), 4.2 (4H, next to UPy), 3.8-3.3 (4nH, PEG), 3.3 (4H, next to urethane), 3.1 (12H, next to urethane and urea), 2.2 (6H, methyl at UPy), 1.6-1.1 (56H, hexyl and dodecyl spacer) ppm.

### Hydrogel formulation

The liquid precursors solutions were prepared by dissolving the polymer powder in alkaline PBS (pH 11.7) and stirring at 70 °C for several hours until dissolved. For a 3 wt% formulation, 30 mg of polymer was dissolved in 970 µl PBS; for a 4 wt% formulation, 40 mg of polymer powder was dissolved in 960 µl PBS; for a 5 wt% formulation, 50 mg of polymer powder was dissolved in 950 µl PBS; for a 6 wt% formulation, 60 mg of polymer powder was dissolved in 940 µl PBS; for a 7 wt% formulation, 70 mg of polymer powder was dissolved in 930 µl PBS. The pH of the precursor solution was adjusted using 1 M NaOH or 1 M HCl to the values as listed below in Table 1. The ability to form a self-supporting hydrogel was assessed by performing the vial inversion test.

### Rheology

Rotational rheology measurements were performed on an Anton Paar Physica (MCR 501) using a conical (CP-50) plate geometry, with a fixed distance of 0.101 mm. For viscosity measurements, shear viscosity was recorded as function of shear rate (500 to 0.1 s⁻¹; 10 points per decade; 38 measuring points; varying measuring point duration log 10→1). At lower shear rates (0.1-1 s⁻¹), low torques were measured resulting in unreliable data. 600 µL of liquid hydrogel solution was applied onto the plate of the rheometer. The viscosity of the different materials was measured at 20°C and 37°C.

### Nebulization of polymer solutions

The suitability of the liquid hydrogel precursors to be nebulized by a clinically relevant PIPAC setup was assessed by laser diffraction (Braet et al.). The same laparoscopic spray nozzle applied in PIPAC surgeries (Capnopen^{®}, Capnomed, Zimmern, Germany) connected to a polyethylene extension line (Vygon, Ecouen, France) and a high-pressure injector (Injektron^{™} 82CT, Medtron, Saarbrücken, Germany) were utilized for high pressure nebulization. The solutions were tested at 20 and 37 °C. Nebulization was performed at clinically relevant conditions, more specifically a maximal upstream injection pressure of 20 bar and a flow rate of 0.7 to 0.8 mL s⁻¹. Unsuccessful aerosol formation was defined as a continuous jet stream of polymer solution exiting the spray nozzle. Successful aerosol formation was defined as a divergent spray of fine droplets exiting the spray nozzle resulting in the formation of a small mist cloud (aerosol).

### 1.2 Results

The ability of the liquid hydrogel solutions to be nebulized is dependent on the resistance to flow, the viscosity. The viscosity of the solutions depends on the length of UPy-PEG polymer, the polymer density, pH and temperature.

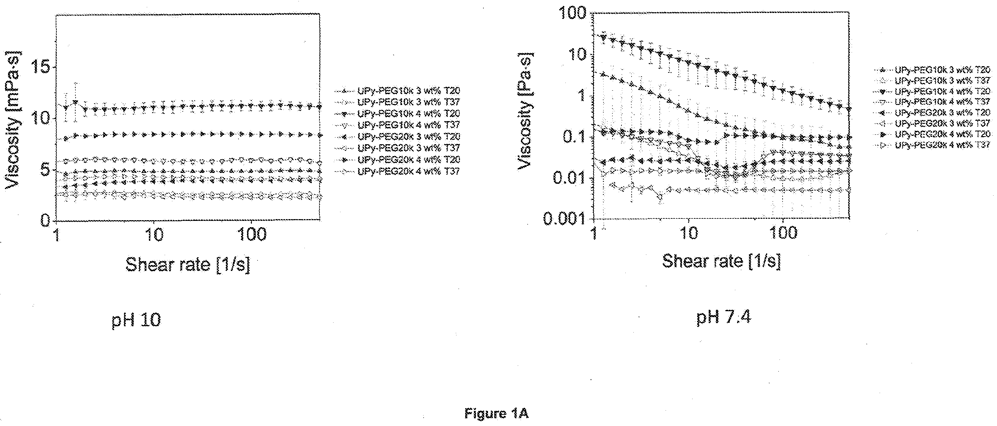

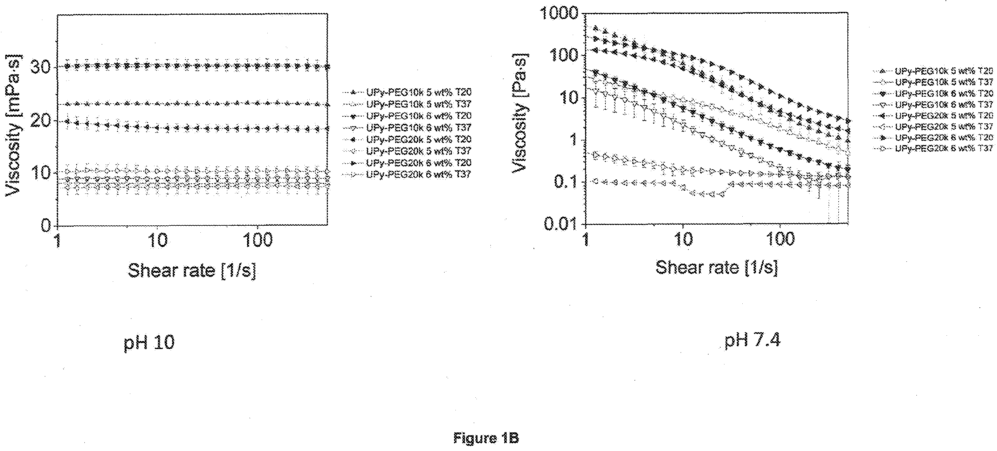

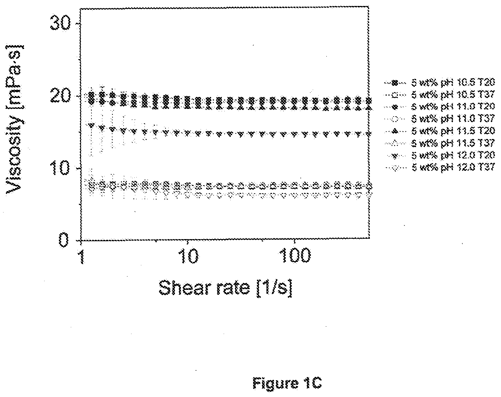

The viscous properties as a function of shear rate of UPy-PEG10k and UPy-PEG20k formulations at 20 and 37 °C are plotted in Figures 1A, 1B and 1C. Figure 1A: 3 and 4 wt%, pH 10 and 7.4. Figure 1B 5 and 6 wt% and pH 10 and 7.4. Figure 1C 5 wt% and pH 10.5, 11.0, 11.5 and 12.0.

The ability of the formulations to form a gel at neutral pH depends on UPy-PEG polymer length, density and temperature. At an elevated pH of 10.0, all liquid solutions display Newtonian behavior with a constant viscosity for all shear rates. At physiological pH, most gels (Gel 1, 2, 5-8) display non-Newtonian behavior with an increase in viscosity at lower shear rates. The increase in viscosity, and resistance to flow, is the result of hydrogel network formation.

Table 1 lists the viscosity at a shear rate of 1/s for the various UPy-PEG formulations and the ability to form an aerosol or hydrogel according to the tests specified above. The optimal formulations that combine the ability to form an aerosol and a stable hydrogel are the 3 and 4 wt% UPy-PEG₁₀ₖ formulations (Gel 1 and 2). UPy-PEG₁₀ₖ formulations with higher densities (5 and 6 wt%; Gel 5 and 6) form more stable hydrogels and display non-Newtonian behaviour with higher viscosities at physiological pH, but their viscosity at pH 10.0 is too high (>25 mPa·s) to enable nebulization. UPy-PEG₂₀ₖ formulations with a similar polymer density (Gel 3 and 4) have sufficiently low viscosities to enable nebulization, but do not form stable gels at physiological pH and display Newtonian fluid behavior.

**Table 1. Rheological properties and aerosolability of various UPy-PEG formulations**

| **Gel** | **Polymer** | **Density** | **pH** | **Viscosity @ 20°C** | **Viscosity @ 37°C** | **Aerosol @ 20°C** | **Gel (vial inversion neutral pH, 20 °C)** |
|---|---|---|---|---|---|---|---|
| 1 | UPy-PEG₁₀ₖ | 3.0 wt% | 10.0 | 5 mPa·s | 3 mPa·s | YES | YES |
| 2 | UPy-PEG₁₀ₖ | 4.0 wt% | 10.0 | 11 mPa·s | 5 mPa·s | YES | YES |
| 3 | UPy-PEG₂₀ₖ | 3.0 wt% | 10.0 | 4 mPa·s | 2 mPa·s | YES | NO |
| 4 | UPy-PEG₂₀ₖ | 4.0 wt% | 10.0 | 8 mPa·s | 4 mPa·s | YES | Viscous liquid |
| 5 | UPy-PEG₁₀ₖ | 5.0 wt% | 10.0 | 28 mPa·s | 8 mPa·s | NO | YES |
| 6 | UPy-PEG₁₀ₖ | 6.0 wt% | 10.0 | 30 mPa·s | 8 mPa·s | NO | YES |
| 7 | UPy-PEG₂₀ₖ | 5.0 wt% | 10.0 | 18 mPa·s | 7 mPa·s | NO | YES |
| 8 | UPy-PEG₂₀ₖ | 6.0 wt% | 10.0 | 30 mPa·s | 10 mPa·s | NO | YES |

To enable the nebulization of more viscous solutions, such as the 5 wt% UPy-PEG₁₀ₖ formulation, the pH can be elevated to lower the number of UPy-dimer interactions and decrease the viscosity, see Figure 1C.

### 1.3 Conclusion

The optimal formulations that combines the ability to form an aerosol and a stable hydrogel is the 4 wt% UPy-PEG₁₀ₖ formulation.

### Example 2 Aerosol formulations of chain-extended UPy-PEG polymers

### 2.1 Materials and methods

### Polymer design & synthesis

The tested UPy-PEG polymers were chain-extended polymers of two different lengths.

They were synthesized as follows:
Telechelic hydroxy terminate poly(ethylene glycol) with a molecular weight of 20 kDa (20.0 gram, 1.0 mmol) was dried at 120 °C *in vacuo* for 2 hours. Subsequently, 5(2-hydroxyethyl)-6-methyl isocytosine) (338 mg, 2.00 mmol), hexanediisocyanate (1.01 gram, 3.00 mmol), 50 mL dimethylformamide and one drop of dibutyltindilaurate were added to the polymer. The reaction mixture was stirred for 12 hours at 90 °C. Subsequently, the reaction mixture was diluted with 50 mL of methanol and poured into 500 mL of diethyl ether. The precipitated polymer was dissolved in 70 mL chloroform and 70 mL methanol and poured into 500 mL diethyl ether. The precipitated polymer was dried *in vacuo* and obtained as a white solid. Chain-extended polymer 1: SEC (DMF/LiBr, PS-standards): M_{w} = 120 kDa, Mn = 88 kDa; Chain-extended polymer 2: SEC (DMF/LiBr, PS-standards): M_{w} = 61 kDa, Mn = 38 kDa

### Hydrogel formulation

The liquid hydrogel precursors were prepared by dissolving the polymer powder in alkaline PBS (pH 11.7) and stirring at 70 °C for several hours until dissolved. For a 4 wt% formulation, 40 mg of polymer powder was dissolved in 960 µl PBS; for a 5 wt% formulation, 50 mg of polymer powder was dissolved in 950 µl PBS; for a 6 wt% formulation, 60 mg of polymer powder was dissolved in 940 µl PBS; The pH of the precursor solution was adjusted using 1 M NaOH or 1 M HCl. The ability to form a self-supporting hydrogel was assessed by performing the vial inversion test. In this test,1 ml of the hydrogel is transferred to a 5 ml vial and its physical state is observed by tube inversion and then it is observed whether the sample flows under its own weight. The ability to be nebulized was screened by applying the hydrogel solution through a 2.3 mm spray catheter (MTW, Wesel, Germany) as a model for an aerosol device.

### Rheology

Rotational rheology measurements were performed on an Anton Paar Physica (MCR 501) using a conical (CP-50) plate geometry, with a fixed distance of 0.101 mm. For viscosity measurements, shear viscosity was recorded as function of shear rate (500 to 0.1 s⁻¹; 10 points per decade; 38 measuring points; varying measuring point duration log 10→1). At lower shear rates (0.1-1 s⁻¹), low torques were measured resulting in unreliable data. 600 µL of liquid hydrogel solution was applied onto the plate of the rheometer. The viscosity of the different materials was measured at 20°C and 37°C.

### 2.2 Results

The measured viscous properties of the two different polymers are listed in Table 2. The viscosity of the liquid hydrogel solution was dependent on polymer density, pH and temperature, similar to example 1. Another important factor is the length of the polymer chain, indicated by the molecular weight (MW). The polymer with high MW (chain extended polymer 1) produced a more viscous solution, already surpassing 10 mPa·s at low polymer density and high pH and temperature, due to increased entanglement of the polymer chains. As a result the solutions could not be applied with the spray catheter. The shorter polymer (chain extended polymer 2) yields lower viscosities and can be applied with the spray catheter using 4 wt% and 5 wt% formulations with pH 12, but not the 6 wt%. The 4 wt% formulation however is unable to form a self-supporting hydrogel at neutral pH. The 5 wt% is able to form a self-supporting hydrogel at neutral pH and is therefore the optimal candidate.

**Table 2 Viscosity of a two chain-extended UPy-PEG polymers at various polymer densities, pH and temperatures.**

| | | **Chain extended polymer 1** | | | |
|---|---|---|---|---|---|
| **Density** | **pH** | **Viscosity @ 20°C [mPa·s]** | **Viscosity @ 37°C [mPa·s]** | **Aerosol @ 20°C** | **Gel (vial inversion neutral pH, 20 °C)** |
| 4 wt% | 10 | 20 | 12 | | |
| | 11 | 13 | 8 | | |
| | 12 | 12 | 8 | **NO** | **YES** |
| 5 wt% | 10 | 32 | 17 | | |
| | 11 | 21 | 12 | | |
| | 12 | 20 | 10 | **NO** | **YES** |
| 6 wt% | 10 | 40 | 22 | | |
| | 11 | 30 | 19 | | |
| | 12 | 26 | 15 | **NO** | **YES** |

| | | **Chain extended polymer 2** | | | |
|---|---|---|---|---|---|
| **Density** | **pH** | **Viscosity @ 20°C [mPa·s]** | **Viscosity @ 37°C [mPa·s]** | **Aerosol @ 20°C** | **Gel (vial inversion neutral pH, 20 °C)** |
| 4 wt% | 10 | 11 | 6 | | |
| | 11 | 9 | 5 | | |
| | 12 | 8 | 5 | **YES** | **NO** |
| 5 wt% | 10 | 19 | 11 | | |
| | 11 | 14 | 8 | | |
| | 12 | 13 | 7 | **YES** | **YES** |
| 6 wt% | 10 | 31 | 18 | | |
| | 11 | 19 | 12 | | |
| | 12 | 13 | 17 | **NO** | **YES** |

### 2.3 Conclusion

Liquid precursor solutions based on chain-extended UPy-PEG polymers can be aerosolized if the molecular weight of the polymer is short, yielding low viscosity solutions. The formulation can be tuned further to combine aerosol behavior at high pH and hydrogel formation at neutral pH.

### Example 3 Droplet size analysis of aerosolized polymer solutions

### 3.1 Materials and methods

### Polymer design

The tested UPy-PEG polymers were the bifunctional telechelic UPy-PEG₁₀ₖ and UPy-PEG₂₀ₖ polymers of Example 1.

### Hydrogel formulation:

The liquid precursor solutions were prepared by dissolving the polymer powder in alkaline PBS (pH 11.7) and stirring at 70 °C for several hours until dissolved. For a 3 wt% formulation, 30 mg of polymer was dissolved in 970 µl PBS; for a 4 wt% formulation, 40 mg of polymer powder was dissolved in 960 µl PBS; for a 5 wt% formulation, 50 mg of polymer powder was dissolved in 950 µl PBS; for a 6 wt% formulation, 60 mg of polymer powder was dissolved in 940 µl PBS; for a 7 wt% formulation, 70 mg of polymer powder was dissolved in 930 µl PBS. The pH of the precursor solution was adjusted to 10 using 1 M NaOH or 1 M HCl.

### Nebulization of polymer solutions for particle size determination

The suitability of the liquid hydrogel precursors to be nebulized by a clinically relevant PIPAC setup was assessed by laser diffraction as described before (Braet et al.). The same laparoscopic spray nozzle applied in PIPAC surgeries (Capnopen^{®}, Capnomed, Zimmern, Germany) connected to a polyethylene extension line (Vygon, Ecouen, France) and a high-pressure injector (Injektron^{™} 82CT, Medtron, Saarbrücken, Germany) were utilized for high pressure nebulization. The solutions were tested at 20 and 37 °C. Nebulization was performed at clinically relevant conditions, more specifically a maximal upstream injection pressure of 20 bar and a flow rate of 0.7 to 0.8 mL s⁻¹.The volume weighed particle size distribution (PSD) of nebulized solutions was analyzed by a Mastersizer S long bench laser diffraction particle size analyzer (Malvern Instruments, Malvern, UK). The tip of the spray nozzle was positioned perpendicularly at a distance of 35 mm to the laser beam and 100 mm to the lens. The aerosol PSD was quantified as previously described in an open laser beam (water vs. air, refractive index of 1.33 and 1.00 respectively) using a 300F lens (0.5-900 µm) over a time period starting at 10 s and ending at 20 s after initiating the nebulization (Van de Sande et al. (1)). The aerosol PSD of nebulized polymer solutions was compared with the aerosol PSD of nebulized physiological saline solution. Results are reported as the volume median diameter (*D*(*v,0.5*)) ± standard deviation.

### 3.2 Results

The capacity of the polymer solutions to be nebulized by a clinically relevant setup was assessed by laser diffraction analysis, through quantification of the aerosol droplet size. Table 3 lists the the volume median diameter (*D*(*v,0.5*)*)* of aerosol droplets formed after atomization of UPy-PEG solution, which were above the droplet size of physiological saline. With regard to polymer density, concentrations higher than the mentioned were also tested, but these solutions exited the nozzle as a jet stream rather than an aerosol or clogged the nozzle.

**Table 3. Measured aerosol droplet size**

| **Formulation** | ***D(v,0.5)* [µm]** |
|---|---|
| Saline | 48 ± 7 |
| UPy-PEG₁₀ₖ 3 wt% pH 10 | 91 ± 17 |
| UPy-PEG₁₀ₖ 4 wt% pH 10 | 94 ± 18 |

### 3.3 Conclusion

Polymer solutions that could be nebulized have an aerosol droplet size in the same order of magnitude as saline.

### Example 4 Mechanical properties before and after aerosolization

### 4.1 Materials and methods

### Hydrogel formulation

The tested hydrogel was the 4 wt% UPy-PEG₁₀ₖ in PBS with a pH of 7.4

### Rheology

Oscillatory rheology measurements were performed on an Anton Paar Physica (MCR 501) using a conical (CP-50) plate geometry, with a fixed distance of 0.101 mm. For strain sweep measurements, storage and loss moduli were recorded as function of strain (0.1-100 %, 10 points per decade, frequency = 1 Hz). For frequency sweep measurements, storage and loss moduli were recorded as function of frequency (0.1-100 rad/s, 10 points per decade, strain = 1%). 600 µL of hydrogel was applied onto the plate of the rheometer. The storage and loss moduli were measured in triplicate at physiological temperature, 37°C.

### 4.2 Results

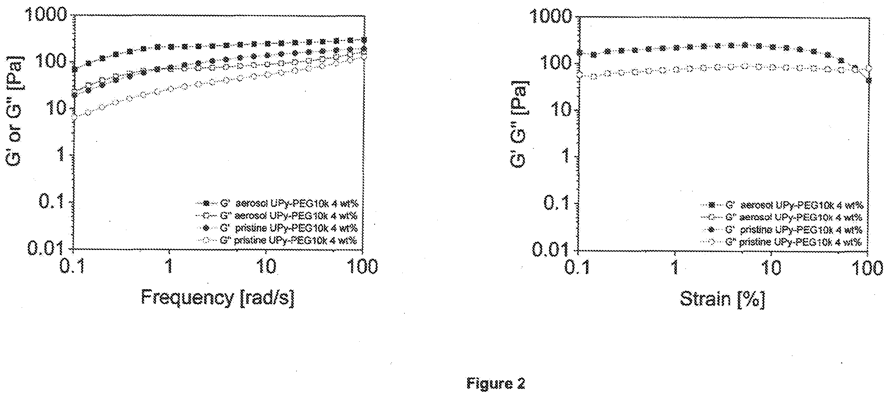

To ensure that gelation properties were not altered by the nebulization procedure, dynamic rheological measurements were executed before and after nebulization. Nebulization was performed with a hydrogel solution with pH 10. For rheology, the pH of the hydrogel was set at 7.4.The viscoelastic characteristics of a 4 wt% UPy-PEG hydrogel prior and following nebulization were measured in function of strain at 37 °C (Figure 2). In all case, storage modulus was higher than the loss modulus demonstrating that a solid material was formed. In the strain sweep experiment, G' and G" completely overlapped before and after nebulization, whereas in the frequency sweep there is a small difference (within the error, errors bars not show) at low frequencies. The results clearly demonstrate that the hydrogel-forming capacity was not compromised by nebulization. The relatively low moduli indicates that a soft material is formed.

### 4.3 Conclusion

Mechanical properties were not compromised by nebulization.

### Example 5 Swelling and degradation of UPy-PEG hydrogels

### 5.1 Materials and methods

### Hydrogel formulation

The tested hydrogel was the 4 wt% UPy-PEG₁₀ₖ in PBS with a pH of 7.4.

### Swelling experiment

The stability and degradation of the UPy-PEG hydrogel in PBS and in a clinically more relevant medium, i.e. peritoneal fluid, was investigated by determining the equilibrium weight swelling ratio (ESR) which represents the hydrogel weight increase caused by water absorption (Equation 1).

7 µL HCl (1 M) was added to 500 µL UPy-PEG solution (4 wt%) to initiate polymerization after which the hydrogel was left to cure for 1 h. Next, 3.0 mL of PBS (pH 7.4) or peritoneal fluid was added to the hydrogel samples which were left to incubate at 37 °C. At predetermined time points, the swelling medium was removed and the residual gels were weighed. The ESR was calculated by comparing the swollen weight of the hydrogel samples (Ws) with their initial dry weight (Wd) following Equation 1: $ESR = \left(Ws-Wd\right) / Wd$ (1)

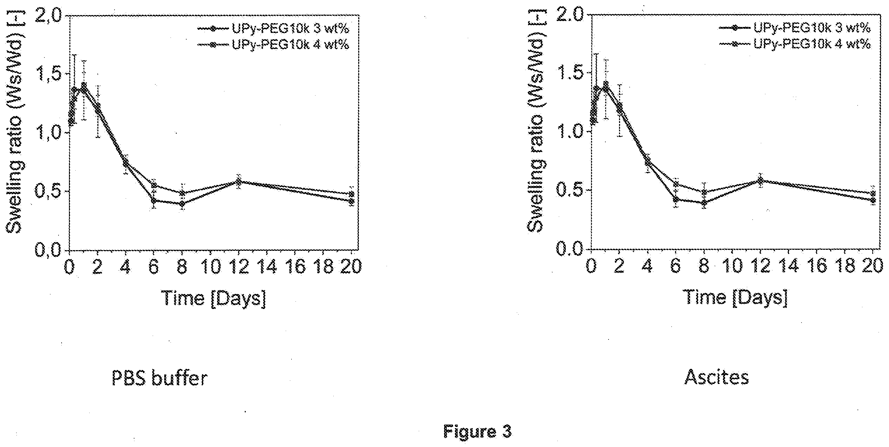

The results are shown in Figure 3.

### 5.2 Results

In order to function as a drug depot for the sustained release of drug compounds, the hydrogels need to remain stable in physiological media for hours or days. In order to study their degradation, the hydrogels were exposed to PBS-buffer to simulate healthy physiological fluid, or ascites, a pathological fluid associated with cancer and their swelling ratio was recorded. In the first hours, UPy-PEG hydrogels slowly take up water and swell. After the second day, the materials rapidly lose mass through erosion of the supramolecular building blocks and after 4-5 days most of the material has degraded.

### 5.3 Conclusion

UPy-PEG hydrogels remain stable for at least two days after which the material starts to degrade by erosion of the polymer chains.

### Example 6 Biocompatibility of aerosol UPy-PEG hydrogel

### 6.1 Materials and methods

### Hydrogel formulation

For the *in vitro* tests, the tested hydrogels were the 3, 6 and 9 wt% UPy-PEG in PBS with a neutral pH after applying the hydrogel to the cell culture insert. For the *in vivo* tests, the tested hydrogel was the 4 wt% UPy-PEG in PBS with a pH of 10.0

### In vitro toxicity

SKOV-3 IP2 cells were cultured at 37 °C in a 95% air/5% CO₂ humidified atmosphere and in McCoy's 5A medium (Invitrogen, Carlsbad, USA) supplemented with 10% fetal bovine serum (FBS) (Invitrogen) and 2% penicillin/streptomycin (Invitrogen).

The *in vitro* viability of SKOV-3 IP2 cells in the presence UPy-PEG (3, 6 and 9% wt) hydrogels was compared to non-treated cells by means of the thiazolyl blue tetrazolium bromide (MTT) assay in function of time. Liquid precursor solutions (100 µL) were transferred to cell culture inserts (Greiner Bio-One, Kremsmünster, Austria) with a membrane mesh size of 8 µm and transformed to hydrogels through the addition of a droplet of 1 M HCl. Cells were seeded in 500 µL medium in 24-well plates (VWR, Radnor, USA) at 50 000, 40 000 and 30 000 cells per well for incubation during respectively 1, 2 and 4 days. 24 h after seeding, cells were washed with PBS and 500 µL fresh culture medium was added to each well. Then, the gel-loaded inserts were placed directly above the seeded cells and left to incubate at 37 °C and 5% CO₂ for 1, 2 and 4 days. After the required incubation time, cells were washed twice with PBS and 500 µL freshly prepared MTT solution (1 mg ml⁻¹ in 50:50 PBS:culture medium) (Sigma Aldrich, Saint Louis, USA) was added to each well for incubation in the dark at 37 °C and 5% CO₂ during 2 h. Next, the MTT solution was removed and 500 µL dimethylsulfoxide (DMSO) was added to dissolve formazan crystals under stirring in the dark for 30 min. Afterwards, 200 µL of solution from each well was transferred to a clear 96-well plate (VWR) and absorbance was measured at 590 nm, normalizing with a reference wavelength of 690 nm using a Victor 3 microplate reader (PerkinElmer, Waltham, USA).

### In vivo toxicity

The *in vivo* compatibility of the UPy-PEG hydrogel was determined by injecting 10 mL of the polymer solution or physiological saline (0.9% NaCl) as negative control in the right lower quadrant of the peritoneal cavity of Wistar Han rats. UPy-PEG (4% wt) solutions were injected at 37 °C and pH 10. The general behavior (activity, food and water intake and signs of pain) and the body weight of each animal was monitored in function of time. After 2 and 7 days, three animals per group were sacrificed. The peritoneal cavity was exposed and samples of the liver, pancreas, spleen and kidneys were excised to look into the general toxicity, while specimens of the colon, cecum, parietal peritoneum and omentum were taken to study local toxicity. The tissues were fixed in 4% paraformaldehyde in PBS for 24 h at room temperature. After overnight dehydration, the specimens were imbedded in paraffin, sectioned at 4 µm and stained with hematoxylin and eosin (H&E). The tissue sections were analyzed with a BX43 bright field microscope (Olympus, Tokyo, Japan) equipped with a ColorView I digital color camera (Olympus) in a blinded manner by a pathologist.

### 6.2 Results

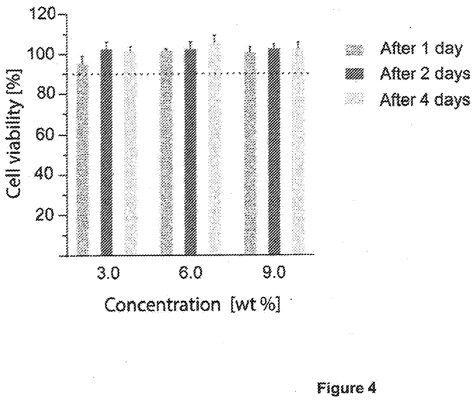

To verify if the stimuli-responsive polymers are biocompatible, the cell viability of SKOV-3 IP2 cells in the presence of UPy-PEG hydrogels was investigated in function of time and concentration. Polymer concentration varied around the optimal concentration for nebulization. As can be seen in Figure 4, for all polymer densities and incubation times, the average cell viability remained above the 90% threshold (dashed line).

Due to the absence of *in vitro* toxicity, these biomaterials were further examined *in vivo* for their biocompatibility, which was evaluated in Wistar Han rats, 2 and 7 days after intraperitoneal injection of the formulations. The general behavior of the animals was not altered and no signs of pain were noted following injection. As an additional measure of the tolerability of the polymer types, the animals' body weight was followed over time. Two days post injection, an average decrease of 5.4 % was detected in the UPy-PEG, respectively. At a later stage, i.e. 7 days after intraperitoneal injection, a weight gain around 5 % was observed again.

Next, histopathological analysis of tissue sections of the pancreas, spleen, liver and kidneys could not reveal any signs of general toxicity of the hydrogel. Regarding the local tolerance of the UPy-PEG hydrogelator, no signs of intolerability were detected in samples of the cecum, parietal peritoneum and colon. At the surface of the omentum, the mesothelial cell layer showed mild, focal inflammation, which was also seen at the adipose tissue surrounding the pancreas.

### 6.3 Conclusion

It is shown that the UPy-PEG hydrogel is non-toxic to cells and that no adverse effects were produced after intraperitoneal administration of the 4 wt% hydrogel.

### Example 7 Uniform distribution of aerosolized UPy-PEG hydrogel

### 7.1 Materials and methods

### Hydrogel formulation

The tested hydrogel was the 4 wt% UPy-PEG₁₀ₖ in PBS with a pH of 10.0.

### In vitro distribution

The gelation and spatial distribution of the nebulized hydrogel was assessed in an *ex vivo* model of the peritoneal cavity which consists of a plexiglass box with a volume of 4 L, comparable to the volume of the human peritoneal cavity. Inside the box, copper plates, which can be heated to 37 °C (Alflex Technologies, Zoetemeer, the Netherlands), are located on the bottom (B), side (S) and top (T). Samples of murine parietal peritoneum were positioned on the heated plates and left to acclimatize for 15 min. After the airtight box was closed, the nozzle was inserted in the box through a gel port (Applied Medical, Amersfoort, The Netherlands), which also serves as a CO2 inlet, enabling the establishment of a pressure of 12 mmHg. Next, nebulization of the polymer solution was initiated by the high-pressure injector which was connected to the nozzle. During nebulization, the same settings and environmental conditions as described above, were applied.

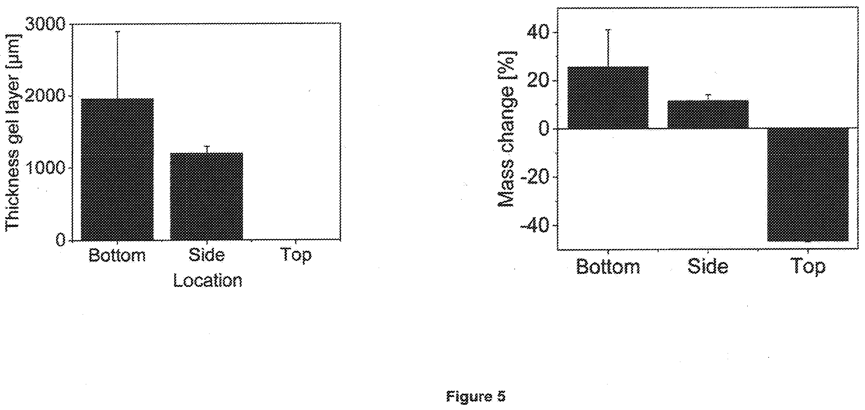

Afterwards, nebulized solutions where left to polymerize for 30 min on the tissue samples in the ex vivo model at 37 °C and 12 mmHg CO2 to allow complete gelation caused by changes in temperature, acidity or shear forces. The gelation time of the hydrogel, which is the time until no flow can be detected in the nebulized solutions, was manually verified. Moreover, the spatial distribution of the hydrogels on the bottom, which is opposite the nozzle, side and top of the ex vivo model was evaluated by recording the mass increase of the peritoneal tissue samples due to hydrogel deposition (Figure 5).

Additionally, the thickness of the gel layer deposited on the tissue specimens was quantified by embedding the gel-coated samples in OCT tissue freezing medium (VWR, Radnor, USA) and cryosectioning them at a diameter of 20 µm using a CM1520 cryostat (Leica). Tissue sections where treated with PBS and Vectashield mounting medium (Vector Laboratories, Burlingame, USA) and mounted between a coverslip n° 1.5 (Menzel-Gläser, Braunschweig, Germany) and microscope glass slide (Thermo Fisher Scientific, Waltham, USA) before imaging took place on a Ti2 inverted microscope combined with an A1R confocal module (Nikon, Tokio, Japan) using a CFI Plan Apo VC 20x air objective, NA 0.75 (Nikon). The transmitted images (TD) were acquired with a LU-N4 laser unit using the 561 nm line, confocal pinhole radius of 17.88 µm, a pixel size of 1.23 µm/pixel and a multi-alkali PMT detector. Images were recorded using the galvano scanner by unidirectional scanning. The scanning speed was set at 15 frames per second (fps), while gain and offset where fixed at respectively 90 and 0. NIS Elements software (Nikon) was applied for imaging. The manual line selection tool in Fiji26 was employed to measure the diameter of the gel layer in threefold at an intermediary distance of 1.5 mm and perpendicular to the tissue surface.

### Animals

Ten to twelve week old female Wistar Han rats (Envigo, Horst, the Netherlands or Janvier, Le Genest-Saint-Isle, France) were kept in standard housing conditions with free access to water and food and a 12 hours light/dark cycle. All animal experiments were approved by the Ethical Committee of the Faculty of Medicine at Ghent University (ECD 20-54). Every surgical procedure was executed under general anesthesia by inhalation of sevoflurane (Abbvie, Lake Bluff, USA) (8 vol% induction, 4 vol% maintenance). When the experimental end point was reached, animals were sacrificed by injection of T-61 (0.3 mL kg-1) (MSD, Kenilworth, USA).

### In vivo distribution

To assess if nebulization leads to a more homogeneous hydrogel distribution in the abdominal cavity compared to injection, Wistar Han rats were intraperitoneally administered with 10 mL of polymer solution, by nebulization and by injection. In order to keep the viscosity of UPy-PEG (4% wt) solution low, they were injected or nebulized at 37 °C and pH 10 and 22 °C. The nebulization procedure was executed as previously described (Van de Sande L. et al. (2); Shariati M. et al.). 5 and 11 mm laparoscopic balloon trocars (Applied Medical, Amersfoort, the Netherlands) were introduced through the abdominal wall, which enabled the insertion of a laparoscope and spray nozzle (Capnopen^{®}, Capnomed, Zimmern, Germany). Next, a capnoperitoneum of 8 mmHg was created by a UHI-3 insufflator (Olympus Surgical Technologies Europe, Hamburg, Germany) and was sustained during the entire procedure. The spray nozzle was connected to a polyethylene extension line (Vygon, Ecouen, France) and a high-pressure injector (Injektron^{™} 82CT, Medtron, Saarbrücken, Germany). Nebulization of the polymer solutions was performed at a maximal upstream injection pressure of 20 bar and a flow rate of 0.8 mL s⁻¹ for the UPy-PEG solution. After 30 min, the capnoperitoneum was deflated and trocars were detached. The incisions were closed using a two-layered running suture (Vicryl Plus 4-0 Ethicon, Johnson & Johnson, New Brunswick, USA). To conclude, buprenorphine (Ceva, Libourne, France) was administered (0.03 mg kg⁻¹, subcutaneously) as analgesic. After 2 and 7 days, three animals per investigational group were sacrificed. The peritoneal cavity was opened by performing a midline laparotomy. The local distribution was visually inspected after nebulization versus injection of the three hydrogels by photographing the exposed abdominal cavity.

### 7.2 Results

The gelation and three dimensional deposition pattern of nebulized hydrogels was assessed in an *ex vivo* model of the human peritoneal cavity under clinically relevant conditions. The gelation time after nebulization of the pH-sensitive UPy-PEG was 15.3 ± 1.5 s. To study the spatial distribution of the nebulized formulation, the thickness of the deposited hydrogel layers was analyzed. The average gel mass thickness on the bottom of the *ex vivo* model, opposite the nozzle, was higher compared to the side position. No hydrogel could be delivered onto samples located at the top of the *ex vivo* model, right behind to the nozzle head. Additionally, recording of the mass change of tissue samples after deposition of the hydrogels led to comparable results (Figure 5).

The efficacy of nebulization as administration technique to create a more homogeneous intraperitoneal hydrogel deposition compared to injection, was evaluated *in vivo.* Both after 2 and 7 days, nebulization of the UPy-PEG solution led to a higher amount of hydrogel which was more evenly distributed across the peritoneal cavity compared to injection, where the formulations mostly remained located at the injection site.

### 7.3 Conclusion

Administration by nebulization resulted in a more uniform UPy-PEG hydrogel distribution than by injection.

### Example 8 Prevention of peritoneal adhesions with aerosolized UPy-PEG hydrogel

### 8.1 Materials and methods

### Hydrogel formulation

The tested hydrogel was the 4 wt% UPy-PEG₁₀ₖ in PBS with a pH of 10.0.

### Peritoneal adhesion model

The capability of the UPy-PEG hydrogel to prevent the formation of postsurgical adhesions was investigated by implementing the abdominal wall and cecum abrasion peritoneal adhesion model in Wistar Han rats as previously described (de Clerck et al., Zhang et al., Ersoy et al.). After a 2 cm midline laparotomy was made, the cecum was exposed and abraded with sterile surgical gauze until petechial hemorrhage occurred. Next, the cecum was relocated to the peritoneal cavity and a 2 x 2 cm defect to the opposite parietal peritoneum was created by rubbing with sterile gauze. Finally, the damaged cecum was put in juxtaposition to the parietal peritoneal wall defect after which the incision was closed using a two-layered running suture (Vicryl Plus 4-0 Ethicon, Johnson & Johnson, New Brunswick, USA). Following adhesion induction, 10 mL of the UPy-PEG gel (4% w/w) or physiological saline (0.9% NaCl) as negative control were administered by nebulization as described above. As a positive control, a commercially available hyaluronic acid based hydrogel (Hyalobarrier^{®}, Anika Therapeutics, Bedford, USA) was applied with a syringe, prior to suturing the incisions, as a thin layer on the abraded cecum and parietal peritoneum according to the manufacturer's instructions. After 2 and 7 days, three animals for each investigational and control group were sacrificed. The peritoneal cavity was exposed by performing a midline laparotomy. The anti-adhesive efficacy of the UPy-PEG hydrogel was compared by implementing two previously described adhesion scoring systems (de Clercq et al., Binda et al, Egea et al.). The first scoring system quantifies the extent, type and tenacity of the adhesions (Table 4) while the second scoring system (Table 5) describes the site of adhesion formation, tenacity and vascularization.

**Table 4. Peritoneal adhesion scoring system 1**

| | | **Score** |
|---|---|---|
| **Extent** | | |
| | No adhesion | 0 |
| | 1-25% of the peritoneal cavity involved | 1 |
| | 26-50% of the peritoneal cavity involved | 2 |
| | 51-75% of the peritoneal cavity involved | 3 |
| | 76-100% of the peritoneal cavity | 4 |
| | involved | |

| **Type** | | |
|---|---|---|
| | No adhesion | 0 |
| | Filmy | 1 |
| | Dense | 2 |
| | Vascular | 3 |

| **Tenacity** | | |
|---|---|---|
| | No adhesion | 0 |
| | Easily fall apart | 1 |
| | Require traction | 2 |
| | Require sharp dissection | 3 |
| **Total adhesion score** | | 0-10 |

**Table 5. Peritoneal adhesion scoring system 2**

| **Site of adhesions** | | |
|---|---|---|
| | Parietal | Pelvic fat body-abdominal wall |
| | | Omentum-abdominal wall |
| | | Intestine-abdominal wall |
| | Visceral | Omentum-liver/stomach/intestine |
| | | Intestine-intestine |
| | | Liver-stomach |

| **Tenacity** | | |
|---|---|---|
| | | |
| | Type 0 | No adhesion |
| | Type 1 | Simple, without dissection |
| | Type 2 | Dissection needed to separate adherent area |
| | Type 3 | Dissection needed to cut the adhesions |

| **Vascularization** | | |
|---|---|---|
| | Vascularized | |
| | Non-vascularized | |

### 8.2 Results

The suitability of the UPy-PEG hydrogel as an anti-adhesive barrier was studied after adhesion induction and hydrogel administration by nebulization. Both parietal and visceral (black adhesions were observed in the Hyalobarrier^{®} and saline groups after 2 and 7 days. UPy-PEG treated animals also presented with parietal and visceral adhesions after 2 days. After 7 days, adhesions could no longer be detected in the UPy-PEG group, demonstrating the anti-adhesive performance of the material. The total adhesion scores that were calculated 2 and 7 days after adhesion induction and hydrogel nebulization are given in Table 6. The UPy-PEG treated animals generated a significantly lower score compared to the saline group. Also, according to the second scoring system, the UPy-PEG group was the only group with solely type 0 adhesions, while type 0 or 3, type 0 or 2 and type 3 adhesions were allocated to both control groups, respectively. Lastly, vascularization of adhesions was identified in Hyalobarrier^{®} and saline groups.

**Table 6. Scoring (according to system 1) of the extent, type, tenacity and total adhesion scores 2 days after adhesion induction and administration of UPy-PEG, Hyalobarrier^{®} and saline. Data are expressed as the average (n=3) ± standard deviation.**

| **Category** | **UPy-PEG** | **Hyalobarrier^{®}** | **Saline** |
|---|---|---|---|
| **Extent** | 0,67 ± 0,58 | 1,00 ± 0,00 | 1,00 ± 0,00 |
| **Type** | 0,67 ± 0,58 | 2,00 ± 0,00 | 2,33 ± 0,58 |
| **Tenacity** | 0,67 ± 0,58 | 2,67 ± 0,58 | 2,67 ± 0,58 |
| **Total** | 2,00 ± 1,73 | 5,67 ± 0,58 | 6,00 ± 1,00 |

**Table 7. Scoring (according to system 1) of the extent, type, tenacity and total adhesion scores 7 days after adhesion induction and administration of UPy-PEG, Hyalobarrier^{®} and saline. Data are expressed as the average (n=3) ± standard deviation.**

| **Category** | **UPy-PEG** | **Hyalobarrier^{®}** | **Saline** |
|---|---|---|---|
| **Extent** | 0,00 ± 0,00 | 2,00 ± 0,00 | 1,33 ± 0,58 |
| **Type** | 0,00 ± 0,00 | 2,67 ± 0,58 | 2,67 ± 0,58 |
| **Tenacity** | 0,00 ± 0,00 | 3,00 ± 0,00 | 3,00 ± 0,00 |
| **Total** | 0,00 ± 0,00 | 7,67 ± 0,58 | 7,00 ± 1,00 |

**Table 8. Scoring (according to system 2) of the site, tenacity and vascularization of adhesions observed 2 days after adhesion induction and administration of UPy-PEG, Hyalobarrier^{®} and saline (n=3 per group).**

| | **UPy-PEG** | **Hyalobarrier^{®}** | **Saline** |
|---|---|---|---|
| **Site adhesions** | | | |
| **Parietal** | 1 | 2 | 2 |
| **Visceral** | 2 | 3 | 2 |

| **Tenacity** | | | |
|---|---|---|---|
| **Type 0** | 1 | 0 | 0 |
| **Type 1** | 2 | 0 | 0 |
| **Type 2** | 0 | 1 | 0 |
| **Type 3** | 0 | 2 | 3 |
| **Vascularization** | 0 | 0 | 2 |

**Table 9. Scoring (according to system 2) of the site, tenacity and vascularization of adhesions observed 7 days after adhesion induction and administration of UPy-PEG, Hyalobarrier^{®} and saline (n=3 per group).**

| | **UPy-PEG** | **Hyalobarrier^{®} Saline** | |
|---|---|---|---|
| **Site adhesions** | | | |
| **Parietal** | 0 | 1 | 2 |
| **Visceral** | 0 | 3 | 2 |

| **Tenacity** | | | |
|---|---|---|---|
| **Type 0** | 3 | 0 | 0 |
| **Type 1** | 0 | 0 | 0 |
| **Type 2** | 0 | 0 | 0 |
| **Type 3** | 0 | 3 | 3 |
| **Vascularization** | 0 | 2 | 2 |

### 8.3 Conclusion

Aerosol UPy-PEG hydrogel is an effective physical barrier to prevent peritoneal adhesions.

### Example 9 Drug release from UPy-hydrogels

### 9.1 Materials and methods

### Hydrogel formulation

The tested hydrogel was the 4 wt% UPy-PEG₁₀ₖ in PBS with pH 7.4.

### PTX-NC formulation

Paclitaxel nanocrystals (PNCs) were prepared by dissolving a stabilizer (Pluronic F127^{®}) in a 20 ml vial containing 5.0 ml of 0.9% NaCl. 50 mg paclitaxel powder (LC Laboratories, Boston, USA) was dispersed in the aqueous phase. Zirconium oxide beads (30 g, diameter 0.5 mm) were added to the suspension as milling pearls. The vials were placed on a roller-mill (Peira, Beerse, Belgium) and grinded at 150 rpm for 60 h. The PNCs were isolated from the grinding pearls by sieving (mesh 180 µm).

### CisPt-NP formulation

CisPt-pArg-HA NPs were prepared by ionic gelation as described before (Shariati et al.). pArg-Cl (MW = 5800 Da) (Alamanda Polymers, Huntsville, USA) was converted to pArg-OH by using an Amberlite IRA 900 Cl ion- exchange resin (1 mL) (Sigma Aldrich, St. Louis, USA), primed with NaOH (1 M, 3 mL). After 30 min, the column was rinsed with MilliQ water until a neutral pH was reached. A pArg-Cl solution (50 mg/mL, 1 mL) was then added to the column, which was subsequently rinsed with 3 mL of MilliQ water to obtain a pArg-OH solution with a concentration of 12.5 mg/mL (pH: 10-12). Before NP preparation, each solution was filtered through a 0.22 µm filter. CisPt-pArg-HA NPs were synthetized by mixing 80 µL pArg-OH solution (2.5 mg/mL) and 120 µL of cisplatin solution (1 mg/mL) (Abcam, Cambridge, UK) in a glass amber vial. Next, 100 µL HA solution (9 mg/mL) (MW = 20 kDa) (Lifecore Biomedical, Chaska, USA) was added at once, after which the NP suspension was kept under stirring for 10 min.

### Drug release experiments

The PNCs and cisPt-NPs were loaded from a stock solution (PNCs: 1.5 mg PTX/mL and cisPt-NPs: 0.250 mg cisPt/mL in solvent) to 0.75 mg PTX/mL and 0.125 mg cisPt/mL in the precursor solution, respectively. The drug loaded solution (200 µL) was transferred to a Millicell insert and converted to a semi-solid hydrogel by the addition of HCl (1 M). Next, the insert was placed in a 24-well plate filled with PBS + 0.2% Tween 80 at pH 7.4 for the PNC loaded gel and PBS at pH 7.4 for the cisPt-NP loaded gel. The plates were incubated at 37 °C and under slow rocking at 50 rpm. At set time points, the release medium was refreshed and the removed medium was analyzed for PTX and cisPt content with UPLC-MS/MS and ICP-MS, respectively. Release experiments were performed with n = 3.

### In vitro cell viability experiment

SKOV-3 IP2 cells were cultured at 37 °C in a 95% air/5% CO₂ humidified atmosphere and in McCoy's 5A medium (Invitrogen, Carlsbad, USA) supplemented with 10% fetal bovine serum (FBS) (Invitrogen) and 2% penicillin/streptomycin (Invitrogen).

The PNCs and cisPt-NPs were loaded from a stock solution (PNCs: 1.5 mg PTX/mL and cisPt-NPs: 0.250 mg cisPt/mL in solvent) to 0.75 mg PTX/mL and 0.125 mg cisPt/mL in the precursor solution, respectively. The drug loaded solutions (100 µL) were transferred to a Millicell insert and converted to a semi-solid hydrogel by the addition of HCl (1 M). As a control, the toxicity of PNC and cisPt-NP loaded hydrogels was compared to the toxicity of PNC and cisPt-NP suspensions with the same concentration, but without the addition of the UPy-PEG polymer. The inserts were placed in a 24-well plate, in which 40 x 10³ SKOV-3 cells per well were seeded 24h prior incubation with the different formulations at 37°C in a 95% air/5% CO₂ humidified atmosphere. At predetermined time points, the inserts were moved to a freshly seeded 24-well plate and further incubated at 37°C in a 95% air/5% CO₂ humidified atmosphere to assess the *in vitro* toxicity of the drug loaded gels in function of time. After the required incubation time, cells were washed twice with PBS and 500 µL freshly prepared MTT solution (1 mg ml⁻¹ in 50:50 PBS:culture medium) (Sigma Aldrich, Saint Louis, USA) was added to each well for incubation in the dark at 37 °C and 5% CO₂ during 2 h. Next, the MTT solution was removed and 500 µL dimethylsulfoxide (DMSO) was added to dissolve formazan crystals under stirring in the dark for 30 min. Afterwards, 200 µL of solution from each well was transferred to a clear 96-well plate (VWR) and absorbance was measured at 590 nm, normalizing with a reference wavelength of 690 nm using a Victor 3 microplate reader (PerkinElmer, Waltham, USA).

### 9.1 Results

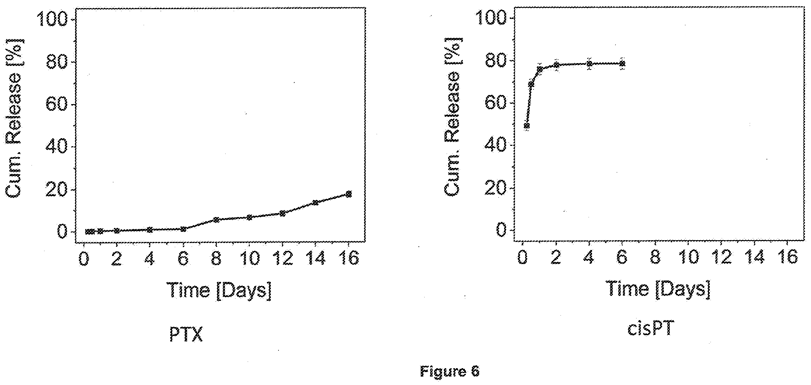

The *in vitro* release profile of hydrogels loaded with hydrophobic PNCs and hydrophilic cisPt-NPs was determined by UPLC-MS/MS and ICP-MS, respectively. Figure 6 shows that the release of PTX from the PNC loaded hydrogel is delayed for 6 days. Next, the cumulative release of PTX steadily increases at each time point, up to approx. 20% after 16 days. Upon hydrogel dissolution, the cumulative release further rises to approx. 50%.

Figure 6 shows a burst release of cisPt from the cisPt-NP loaded hydrogel of approx. 75% after 1 day. Next, the released amount of cisPt per time point steadily decreases, reaching a plateau around 80% of cumulative release after 6 days. Hydrogel dissolution does not seem to have a major influence on the total amount of released cisPt.

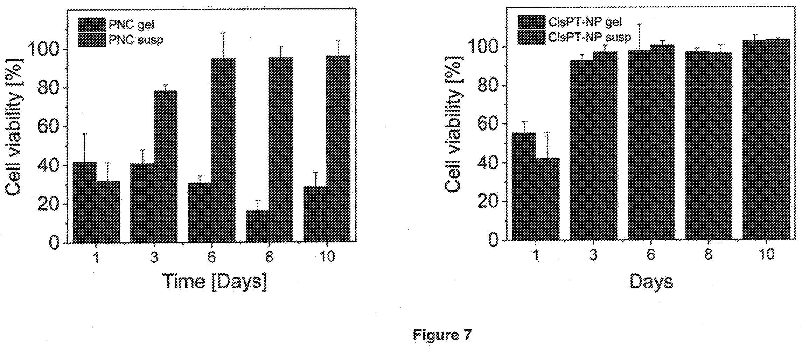

Figure 7 shows the cellular toxicity caused by the PNC loaded hydrogel and PNC suspension. The PNC loaded hydrogel is able to maintain the cytotoxic effect during 10 days, while the *in vitro* toxicity of the PNC suspension starts to decrease after 3 days. This in contrast to the cisPt-NP formulations, where the cytotoxic effect of both the hydrogel and suspension declines after 3 days.

### 9.3 Conclusion

The UPy-PEG hydrogel can serve as an effective carrier which is able to control the release of both hydrophobic (e.g. paclitaxel) and hydrophilic (e.g. cisplatin) drugs. Drug release and cytotoxicity of the hydrogels seem to be linked to the hydrophobicity of the enclosed compounds. More specifically, hydrophobic drugs such as paclitaxel are more slowly released from the hydrogel compared to hydrophilic agents such as cisplatin. Similarly, paclitaxel loaded hydrogels exert a cytotoxic effect during a longer time period compared to cisplatin loaded hydrogels.

### Example 10 - UPy-PEG hydrogel formulations for the sustained delivery of TLR-agonists

### 10.1 Materials and methods

### Hydrogel formulations

The tested hydrogels were UPy-PEG10k as described in Example 1, 6 and 10 wt% in PBS, and chain extended polymer 1 as described in Example 2 (CE-Polymer 1), 6 and 10 wt% in PBS.

### Drug release experiments

The TLR-agonist resiquimod (synonym R848) was loaded from a stock solution (50 mM in DMSO) to 1.27 mM in the hydrogel. The TLR-agonist telratolimod (synonym MEDI9197) was loaded from a stock solution (10 mM in DMSO) to 0.5 mM in the hydrogel.

The drug loaded solution (100 µL) was transferred to a Millicell insert, which was placed in a 24-wells plate filled with PBS pH 7.4 (700 µL). At set time points the PBS was refreshed and the removed PBS was analyzed for R848 content UV absorbance at 320 nm. For MEDI9197, PBS was substituted for a volatile ammonium carbonate buffer and a HPLC-MS method was used. Release experiments were performed with n = 3.

### 10.2 Results

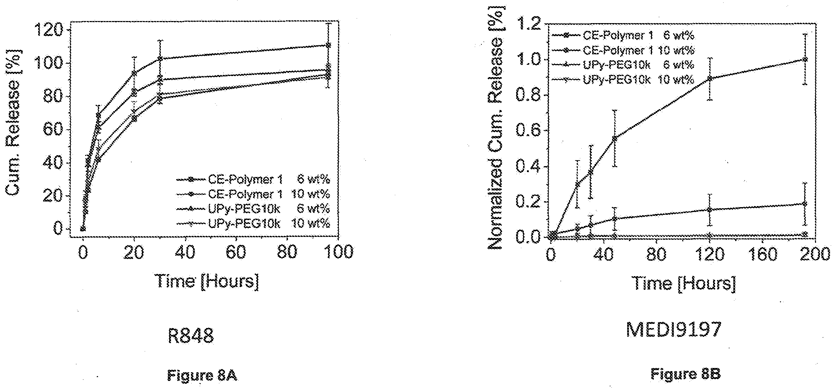

*In vitro* drug release experiments using hydrogel-drug formulations of two different TLR-agonists (R848 and MEDI9197) were performed to produce a good understanding of the molecular interactions between drug compounds and the hydrogel network. Both compounds are TLR-agonists that are potent activators of the immune system by converting the immunosuppressive environment of tumors into an immunoactive environment through cytokine secretion and activation of cytotoxic lymphocytes. TLR-agonists can remodel the tumor microenvironment and reprogram the host response, promoting antitumor immunity. However, the widespread application of TLR-agonists is hampered by severe toxic side effects caused by systemic and nonspecific immune responses leading to a cytokine storm. To overcome this problem, delivery of TLR-agonists needs to be localized to the disease area (e.g. peritoneal cavity) to improve the safety and effectiveness of this immune therapy. TLR-agonist R848 is relatively hydrophilic and most of the drug is released within 24 hours (Figure 8A). TLR-agonist MEDI9197 is relatively hydrophobic due to due to its long alkyl tail. Due to the hydrophobic nature of the molecule and consequent affinity with the hydrophobic compartments of the hydrogel, MEDI9197 is released slower than MMC (Figure 8B). Especially UPy-PEG10k hydrogel are able to stably retain MEDI9197 (lower curves in Figure 8B). The different release kinetics of these TLR-agonists can be used to control the timing of, and localize the therapeutic effect of the immune system activating drugs and improve safety and effectiveness.

### 10.3 Conclusion

UPy-PEG hydrogels can be used as effective formulation vehicles for the local and sustained release of TLR-agonists.

### Example 11 - UPy-PEG hydrogel formulations for the sustained delivery of monoclonal antibodies

### 11.1 Materials and methods

### Hydrogel formulations

The tested hydrogels was UPy-PEG10k as described in Example 1,10 wt% in PBS.

### Drug release experiments

The monoclonal antibody ipilimumab was loaded from a stock solution (5.0 mg/ml in PBS) to 3 µg/µl in the hydrogel. The drug loaded solution (50 µL) was transferred to 96-wells filter plate filled with PBS pH 7.4 (250 µL). At set time points the PBS was refreshed and the removed PBS was analyzed for antibody content with a Fortebio Octet interferometer.

### 11.2 Results

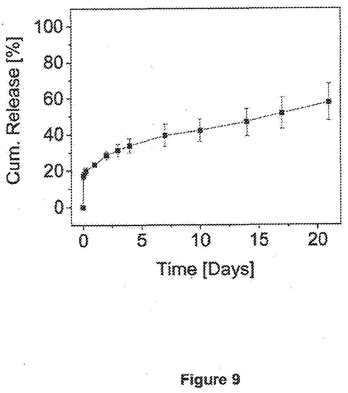

The results are shown in Figure 9.

Ipilimumab is checkpoint inhibitor (CTLA-4) and binds to the protein CTLA-4 on cytotoxic T lymphocytes. This leads to blocking the inhibitory signal that is exploited by tumor cells to downregulate the tumoricidal activity of the immune system. As a result cytotoxic T lymphocytes are robustly activated to kill tumor cells.

Immune checkpoint inhibitors such as ipilimumab are known to have limited accumulation and retention at the tumor site. Also due to their low specificity, normal tissues can be affected and serious autoimmune diseases are produced. Therefore effective drug delivery methods and vehicles are needed to increase the efficacy of immunotherapy. The hydrogel formulation described in this example is a suitable vehicle for the improved local and prolonged delivery of immune checkpoint inhibitors as the antibodies is released for multiple weeks. In contrast to small molecules, the retention of the antibodies is not governed by hydrophobic interactions but occurs as a result of limited diffusion due to the size of the molecule (~150 kDa molecular weight, 12 nm hydrodynamic radius)

### 11.3 Conclusion

UPy-PEG hydrogels can be used as effective formulation vehicles for the local and sustained release of large molecules such as monoclonal antibodies.

## Claims

1. An aerosol composition comprising:
a) a UPy-PEG polymer having hydrophobic hard blocks covalently bonded with hydrophilic soft blocks wherein the hydrophobic hard blocks comprise 2-ureido-4[1H]-pyrimidinone (UPy) moieties and aliphatic spacers and the hydrophilic soft blocks comprise polyethylene glycol moieties; and
b) a pharmaceutically acceptable carrier
wherein the aerosol composition is **characterized by**
• a viscosity of 1 to 500 mPa.s, preferably 1 to 100 mPa.s over a temperature range of 15 to 25 °C and a pH range of 8.5 to 14;
• a viscosity of 1 to 20 mPa.s, preferably 1 to 5 mPa.s over a temperature range of 35 to 50 °C and a pH range of 8.5 to 14;
• a viscosity of at least 100 mPa.s, preferably at least 1000 mPa.s over a temperature range of 35 to 40 °C and a pH range of 6 to 8,
wherein the viscosity is measured at shear rate 1 s⁻¹,
for use in a method of treatment or prevention of a disease of the peritoneal cavity, wherein the treatment comprises administration of the aerosol composition to the peritoneal cavity by nebulization.

2. The aerosol composition for use according to claim 1, wherein the aerosol composition has a storage modulus of 10 to 2000 Pa at a temperature range of 35 to 40 °C.

3. The aerosol composition for use according to claims 1 or 2, wherein the pharmaceutically acceptable carrier is an aqueous solution comprising water and optionally a buffer.

4. The aerosol composition for use according to any one of claims 1 to 3, wherein the concentration of the UPy-PEG polymer in the pharmaceutically acceptable carrier is 0.5 to 20 wt.%, preferably 1 to 10 wt.%, more preferably 4 to 8 wt.%, based on the total weight of the aerosol composition.

5. The aerosol composition for use according to any one of claims 1 to 4, which has a pH of at least 9, preferably of 10 to 12.

6. The aerosol composition for use according to any one of claims 1 to 5, wherein the UPy-PEG polymer is obtainable by the reaction of:
a compound having formula (I) 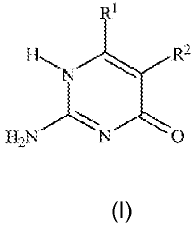 wherein
R¹ is independently selected from the group consisting of hydrogen and C₁-C₂₀ alkyl,
R² is independently selected from the group consisting of hydrogen and C₁-C₂₀ alkyl,
wherein C₁-C₂₀ alkyl is optionally substituted by -OH,
a diisocyanate compound with formula OCN-R³-NCO
wherein R³ is C₂-C₁₆ alkyl or C₄-C₁₆ alkenyl
optionally, an aliphatic spacer which is C₂-C₂₄ alkyl
and a polyethylene glycol.

7. The aerosol composition for use according to any one of claims 1 to 6, wherein the UPy-PEG polymer has a ratio of hydrophobic hard blocks to hydrophilic soft blocks of 1:5 to 1:25 based on molecular weight.

8. The aerosol composition for use according to any one of claims 1 to 7, wherein the UPy-PEG polymer comprises hydrophilic polyethylene glycol soft blocks having a molecular weight Mₙ of 5,000 to 30,000 Da, preferably 10,000 to 20,000 Da.

9. The aerosol composition for use according to any one of claims 1 to 8, wherein the UPy-PEG polymer has a molecular weight Mₙ of 5,000 to 1,000,000 Da, preferably 10,000 to 100,000 Da, more preferably 10,000 to 50,000 Da.

10. The aerosol composition for use according to any one of claims 1 to 9, further comprising a pharmaceutically active agent.

11. The aerosol composition for use according to any one of claims 1 to 10, wherein the nebulization creates an aerosol with a droplet size of less than 200 µm, preferably less than 150 µm, more preferably less than 100 µm.

12. The aerosol composition for use according to any one of claims 1 to 11, wherein the nebulization is carried out under elevated pressure, preferably using a pressurized intraperitoneal chemotherapy (PIPAC) spray nozzle and a high pressure injector, more preferably using electrostatic precipitation pressurized intraperitoneal chemotherapy (ePIPAC) treatment.

13. The aerosol composition for use according to claim 12, wherein the treatment further comprises inducing an electrostatic field in the peritoneal cavity.

14. The aerosol composition for use according to any one of claims 1 to 13, wherein the treatment further comprises inducing a capnoperitoneum with carbon dioxide gas.

15. The aerosol composition for use according to any one of claims 1 to 14, wherein the disease comprises adhesions in the peritoneal cavity.

16. The aerosol composition for use according to claim 10, wherein the disease comprises peritoneal metastases.

17. The aerosol composition for use according to any one of claims 1 to 16, wherein the nebulization of the aerosol composition creates a gel in the peritoneal cavity that persists *in situ* for at least 6 hours.

18. The aerosol composition for use according to claim 10, wherein the method of treatment comprises sustained release of a pharmaceutically active agent in the peritoneal cavity after administration of the aerosol composition, preferably for at least 6 hours.

19. A drug nebulizing device for use in a method of treatment or prevention of a disease of the peritoneal cavity, wherein the device is adapted for delivery of aerosolized pharmaceutical compositions and wherein the device comprises:
a reservoir containing the aerosol composition according to any one of claims 1 to 18, optionally provided with means to control temperature; and
a spray nozzle; and optionally
means to create high pressurized aerosol; and optionally
means to create electrostatic field.

20. A reservoir containing the aerosol composition according to any one of claims 1 to 18 for use in a drug nebulizing device according to claims 19.

## Patentansprüche

1. Aerosolzusammensetzung, umfassend:
a) ein UPy-PEG-Polymer mit hydrophoben Hartblöcken, die kovalent an hydrophile Weichblöcke gebunden sind, wobei die hydrophoben Hartblöcke 2-Ureido-4[1H]-pyrimidinon(UPy)-Einheiten und aliphatische Spacer umfassen und die hydrophilen Weichblöcke Polyethylenglykoleinheiten umfassen; und
b) einen pharmazeutisch unbedenklichen Träger,
wobei die Aerosolzusammensetzung **gekennzeichnet ist durch**
• eine Viskosität von 1 bis 500 mPa.s, bevorzugt 1 bis 100 mPa.s über einen Temperaturbereich von 15 bis 25 °C und einen pH-Bereich von 8,5 bis 14;
• eine Viskosität von 1 bis 20 mPa.s, bevorzugt 1 bis 5 mPa.s über einen Temperaturbereich von 35 bis 50 °C und einen pH-Bereich von 8,5 bis 14;
• eine Viskosität von mindestens 100 mPa.s, bevorzugt mindestens 1000 mPa.s, über einen Temperaturbereich von 35 bis 40 °C und einen pH-Bereich von 6 bis 8,
wobei die Viskosität bei einer Scherrate von 1 s⁻¹ gemessen wird,
zur Verwendung in einem Verfahren zur Behandlung oder Prävention einer Erkrankung der Peritonealhöhle, wobei die Behandlung Verabreichung der Aerosolzusammensetzung an die Peritonealhöhle durch Vernebelung umfasst.

2. Aerosolzusammensetzung zur Verwendung nach Anspruch 1, wobei die Aerosolzusammensetzung einen Speichermodul von 10 bis 2000 Pa in einem Temperaturbereich von 35 bis 40 °C aufweist.

3. Aerogelzusammensetzung zur Verwendung nach den Ansprüchen 1 oder 2, wobei der pharmazeutisch unbedenkliche Träger eine wässrige Lösung ist, die Wasser und gegebenenfalls einen Puffer umfasst.

4. Aerosolzusammensetzung zur Verwendung nach einem der Ansprüche 1 bis 3, wobei die Konzentration des UPy-PEG-Polymers in dem pharmazeutisch unbedenklichen Träger 0,5 bis 20 Gew.-%, bevorzugt 1 bis 10 Gew.-%, besonders bevorzugt 4 bis 8 Gew.-% beträgt, bezogen auf das Gesamtgewicht der Aerosolzusammensetzung.

5. Aerosolzusammensetzung zur Verwendung nach einem der Ansprüche 1 bis 4, die einen pH-Wert von mindestens 9, bevorzugt von 10 bis 12 aufweist.

6. Aerosolzusammensetzung zur Verwendung nach einem der Ansprüche 1 bis 5, wobei das UPy-PEG-Polymer erhältlich ist durch die Reaktion von:
einer Verbindung mit der Formel (I): 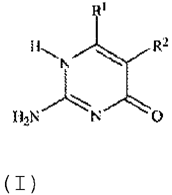
wobei
R¹ unabhängig ausgewählt ist aus der Gruppe bestehend aus Wasserstoff und einem C₁-C₂₀-Alkyl,
R² unabhängig ausgewählt ist aus der Gruppe bestehend aus Wasserstoff und einem C₁-C₂₀-Alkyl,
wobei C₁-C₂₀-Alkyl gegebenenfalls durch -OH substituiert ist,
einer Diisocyanatverbindung mit der Formel OCN-R³-NCO, wobei R³ C₂-C₁₆-Alkyl oder C₄-C₁₆-Alkenyl ist,
gegebenenfalls einem aliphatischen Spacer, der C₂-C₂₄-Alkyl ist,
und einem Polyethylenglykol.

7. Aerosolzusammensetzung zur Verwendung nach einem der Ansprüche 1 bis 6, wobei das UPy-PEG-Polymer ein Verhältnis von hydrophoben Hartblöcken zu hydrophilen Weichblöcken von 1:5 bis 1:25 aufweist, bezogen auf das Molekulargewicht.

8. Aerosolzusammensetzung zur Verwendung nach einem der Ansprüche 1 bis 7, wobei das UPy-PEG-Polymer hydrophile Polyethylenglykol-Weichblöcke mit einem Molekulargewicht Mₙ von 5.000 bis 30.000 Da, bevorzugt 10.000 bis 20.000 Da, umfasst.

9. Aerosolzusammensetzung zur Verwendung nach einem der Ansprüche 1 bis 8, wobei das UPy-PEG-Polymer ein Molekulargewicht Mₙ von 5.000 bis 1.000.000 Da, bevorzugt 10.000 bis 100.000 Da, besonders bevorzugt 10.000 bis 50.000 Da aufweist.

10. Aerosolzusammensetzung zur Verwendung nach einem der Ansprüche 1 bis 9, ferner umfassend ein pharmazeutisch aktives Mittel.

11. Aerosolzusammensetzung zur Verwendung nach einem der Ansprüche 1 bis 10, wobei die Vernebelung ein Aerosol mit einer Tröpfchengröße von weniger als 200 µm, bevorzugt weniger als 150 µm, besonders bevorzugt weniger als 100 µm erzeugt.

12. Aerosolzusammensetzung zur Verwendung nach einem der Ansprüche 1 bis 11, wobei die Vernebelung unter erhöhtem Druck durchgeführt wird, bevorzugt unter Verwendung einer unter Druck stehenden intraperitonealen Chemotherapie(PIPAC)-Sprühdüse und eines Hochdruckinjektors, besonders bevorzugt unter Verwendung einer Behandlung mit unter Druck stehender intraperitonealer Chemotherapie mit elektrostatischer Fällung (ePIPAC).

13. Aerosolzusammensetzung zur Verwendung nach Anspruch 12, wobei die Behandlung ferner Induzieren eines elektrostatischen Feldes in der Peritonealhöhle umfasst.

14. Aerosolzusammensetzung zur Verwendung nach einem der Ansprüche 1 bis 13, wobei die Behandlung ferner Induzieren eines Capnoperitoneums mit Kohlendioxidgas umfasst.

15. Aerosolzusammensetzung zur Verwendung nach einem der Ansprüche 1 bis 14, wobei die Erkrankung Adhäsionen in der Peritonealhöhle umfasst.

16. Aerosolzusammensetzung zur Verwendung nach Anspruch 10, wobei die Erkrankung Peritonealmetastasen umfasst.

17. Aerosolzusammensetzung zur Verwendung nach einem der Ansprüche 1 bis 16, wobei die Vernebelung der Aerosolzusammensetzung ein Gel in der Peritonealhöhle erzeugt, das in situ für mindestens 6 Stunden persistiert.

18. Aerosolzusammensetzung zur Verwendung nach Anspruch 10, wobei das Behandlungsverfahren anhaltende Freisetzung eines pharmazeutisch aktiven Mittels in der Peritonealhöhle nach Verabreichung der Aerosolzusammensetzung umfasst, bevorzugt für mindestens 6 Stunden.

19. Arzneimittelvernebelungsvorrichtung zur Verwendung in einem Verfahren zur Behandlung oder Prävention einer Erkrankung der Peritonealhöhle, wobei die Vorrichtung zur Abgabe aerosolisierter pharmazeutischer Zusammensetzungen eingerichtet ist, und wobei die Vorrichtung Folgendes umfasst:
ein Reservoir, das die Aerosolzusammensetzung nach einem der Ansprüche 1 bis 18 enthält, gegebenenfalls ausgestattet mit Mitteln zur Temperatursteuerung; und
eine Sprühdüse; und gegebenenfalls
Mittel zum Erzeugen von unter hohem Druck stehendem Aerosol; und gegebenenfalls
Mittel zur Erzeugung eines elektrostatischen Feldes.

20. Reservoir, das die Aerosolzusammensetzung nach einem der Ansprüche 1 bis 18 enthält, zur Verwendung in einer Arzneimittelverneblungsvorrichtung nach Anspruch 19.

## Revendications

1. Composition d'aérosol comprenant :
a) un polymère UPy-PEG ayant des blocs durs hydrophobes liés de manière covalente à des blocs souples hydrophiles, dans laquelle les blocs durs hydrophobes comprennent des groupements 2-uréido-4[1H]-pyrimidinone (UPy) et des espaceurs aliphatiques et les blocs souples hydrophiles comprennent des groupements polyéthylène glycol ; et
b) un support pharmaceutiquement acceptable
la composition aérosol étant **caractérisée par**
• une viscosité de 1 à 500 mPa.s, de préférence 1 à 100 mPa.s sur une plage de température de 15 à 25 °C et une plage de pH de 8,5 à 14 ;
• une viscosité de 1 à 20 mPa.s, de préférence 1 à 5 mPa.s sur une plage de température de 35 à 50 °C et une plage de pH de 8,5 à 14 ;
• une viscosité d'au moins 100 mPa.s, de préférence d'au moins 1 000 mPa.s sur une plage de température de 35 à 40 °C et une plage de pH de 6 à 8,
la viscosité étant mesurée à une vitesse de cisaillement de 1 s⁻¹,
pour une utilisation dans un procédé de traitement ou de prévention d'une maladie de la cavité péritonéale, dans laquelle le traitement comprend l'administration de la composition d'aérosol à la cavité péritonéale par nébulisation.

2. Composition d'aérosol destinée à être utilisée selon la revendication 1, dans laquelle la composition d'aérosol a un module de conservation de 10 à 2 000 Pa dans une plage de température de 35 à 40 °C.

3. Composition d'aérosol destinée à être utilisée selon la revendication 1 ou 2, dans laquelle le support pharmaceutiquement acceptable est une solution aqueuse comprenant de l'eau et éventuellement un tampon.

4. Composition d'aérosol pour une utilisation selon l'une quelconque des revendications 1 à 3, la concentration du polymère UPy-PEG dans le support pharmaceutiquement acceptable étant de 0,5 à 20 % en poids, de préférence de 1 à 10 % en poids, plus préférablement de 4 à 8 % en poids, sur la base du poids total de la composition d'aérosol.

5. Composition aérosol destinée à être utilisée selon l'une quelconque des revendications 1 à 4, qui a un pH d'au moins 9, de préférence de 10 à 12.

6. Composition d'aérosol pour utilisation selon l'une quelconque des revendications 1 à 5, le polymère UPy-PEG pouvant être obtenu par la réaction de :
un composé ayant la formule (I) 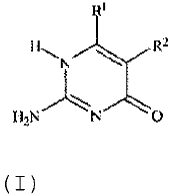
dans laquelle
R¹ est indépendamment choisi dans le groupe constitué par hydrogène et C₁-C₂₀ alkyle,
R² est indépendamment choisi dans le groupe constitué par hydrogène et C₁-C₂₀ alkyle,
dans laquelle alkyle en C₁-C₂₀ est éventuellement substitué par -OH,
un composé diisocyanate ayant une formule OCN-R³-NCO dans laquelle R³ est C₂-C₁₆ alkyle ou C₄-C₁₆ alcényle éventuellement, un espaceur aliphatique qui est alkyle en C₂-C₂₄
et un polyéthylèneglycol.

7. Composition d'aérosol destinée à être utilisée selon l'une quelconque des revendications 1 à 6, dans laquelle le polymère UPy-PEG a un rapport de blocs durs hydrophobes sur blocs souples hydrophiles de 1:5 à 1:25 sur la base du poids moléculaire.

8. Composition d'aérosol destinée à être utilisée selon l'une quelconque des revendications 1 à 7, dans laquelle le polymère UPy-PEG comprend des blocs souples de polyéthylène glycol hydrophiles ayant un poids moléculaire Mₙ de 5 000 à 30 000 Da, de préférence de 10 000 à 20 000 Da.

9. Composition d'aérosol destinée à une utilisation selon l'une quelconque des revendications 1 à 8, le polymère UPy-PEG ayant un poids moléculaire Mₙ de 5 000 à 1 000 000 Da, préférablement de 10 000 à 100 000 Da, plus préférablement de 10 000 à 50 000 Da.

10. Composition d'aérosol pour une utilisation selon l'une quelconque des revendications 1 à 9, comprenant en outre un agent pharmaceutiquement actif.

11. Composition d'aérosol destinée à être utilisée selon l'une quelconque des revendications 1 à 10, dans laquelle la nébulisation crée un aérosol ayant une taille de gouttelettes inférieure à 200 µm, de préférence inférieure à 150 pm, plus préférablement inférieure à 100 µm.

12. Composition d'aérosol destinée à être utilisée selon l'une quelconque des revendications 1 à 11, dans laquelle la nébulisation est effectuée sous pression élevée, de préférence en utilisant une buse de pulvérisation de chimiothérapie intrapéritonéale sous pression (PIPAC) et un injecteur haute pression, plus préférablement en utilisant un traitement de chimiothérapie intrapéritonéale sous pression par précipitation électrostatique (ePIPAC).

13. Composition d'aérosol destinée à être utilisée selon la revendication 12, dans laquelle le traitement comprend en outre l'induction d'un champ électrostatique dans la cavité péritonéale.

14. Composition d'aérosol destinée à être utilisée selon l'une quelconque des revendications 1 à 13, dans laquelle le traitement comprend en outre l'induction d'un capnopéritoine avec du dioxyde de carbone.

15. Composition d'aérosol destinée à être utilisée selon l'une quelconque des revendications 1 à 14, dans laquelle la maladie comprend des adhérences dans la cavité péritonéale.

16. Composition d'aérosol destinée à être utilisée selon la revendication 10, dans laquelle la maladie comprend des métastases péritonéales.

17. Composition d'aérosol destinée à être utilisée selon l'une quelconque des revendications 1 à 16, dans laquelle la nébulisation de la composition d'aérosol crée un gel dans la cavité péritonéale qui persiste *in situ* pendant au moins 6 heures.

18. Composition d'aérosol destinée à être utilisée selon la revendication 10, dans laquelle le procédé de traitement comprend la libération prolongée d'un agent pharmaceutiquement actif dans la cavité péritonéale après administration de la composition d'aérosol, de préférence pendant au moins 6 heures.

19. Dispositif de nébulisation de médicament destiné à être utilisé dans un procédé de traitement ou de prévention d'une maladie de la cavité péritonéale, dans lequel le dispositif est adapté pour l'administration de compositions pharmaceutiques en aérosol et dans lequel le dispositif comprend :
un réservoir contenant la composition d'aérosol selon l'une quelconque des revendications 1 à 18, éventuellement pourvu de moyens pour réguler la température ; et
une buse de pulvérisation ; et éventuellement des moyens pour créer un aérosol sous haute pression ;
et éventuellement des moyens pour créer un champ électrostatique.

20. Réservoir contenant la composition d'aérosol selon l'une quelconque des revendications 1 à 18, destiné à être utilisé dans un dispositif de nébulisation de médicament selon la revendication 19.
